# EUROPÄISCHE PATENTSCHRIFT

(11) **EP 0 662 472 B1**
(45) Veröffentlichungstag und Bekanntmachung des Hinweises auf die Patenterteilung: **11.03.1998**
(21) Anmeldenummer: 94810737.0
(22) Anmeldetag: 20.12.1994
(51) Int. Cl.: C07C 309/65, C07C 309/66, C07D 333/38, C07D 333/70, C07D 233/54, C07C 327/56, C07C 337/08, C07C 337/04, C07D 295/14, C07C 323/60, C07C 333/28, C07C 329/16, C07C 333/20, C07D 295/20, C07D 207/34, C07D 277/50, A01N 33/26

(54) **Hydrazonderivate als Schädlingsbekämpfungsmittel**
Hydrazone derivates as pesticides
Dérivés d'hydrazone comme pesticides

(30) Priorität: 05.01.1994 CH 11/94
(43) Veröffentlichungstag der Anmeldung: 12.07.1995
(73) Patentinhaber: Novartis AG, 4058 Basel (CH)
(72) Erfinder: Dürr, Dr. Dieter, CH-4103 Bottmingen (CH); Hall, Dr. Roger Graham, CH-4147 Aesch (CH); Maienfisch, Dr. Peter, CH-4118 Rodersdorf (CH); Pascual, Dr. Alfons, CH-4053 Basel (CH); Böger, Manfred, D-79576 Weil am Rhein (DE)

(56) Entgegenhaltungen:
- EP-A- 0 003 913
- EP-A- 0 026 040
- EP-A- 0 566 534
- PATENT ABSTRACTS OF JAPAN, vol. 16, no. 140 (C-0926), 8.April 1992 & JP-A-04 001173

## Beschreibung

Die Erfindung betrifft Verbindungen der Formel 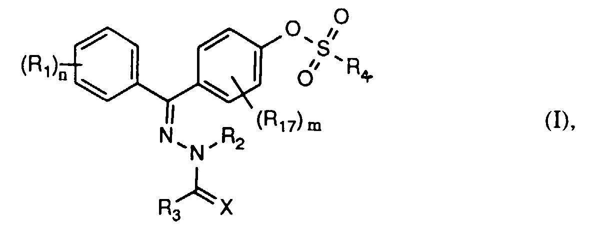 worin
- m: 0, 1, 2, 3 oder 4, wobei, wenn m grösser als 1 ist, die Reste R₁₇ gleich oder verschieden sind;
- n: 0, 1, 2, 3, 4 oder 5, wobei, wenn n grösser als 1 ist, die Reste R₁ gleich oder verschieden sind;
- R₁ und R₁₇: unabhängig voneinander Halogen, C₁-C₈-Alkyl, Halogen-C₁-C₈-Alkyl, Phenoxy, Phenylthio, Halogen-C₁-C₈-Alkylthio, Phenyl, C₁-C₈-Alkylthio, C₁-C₈-Alkylcarbonyl, OSO₂R₄, SO₂NR₁₃R₁₄ oder P(=Y)R₂₂R₂₃;
entweder X S, R₂ R₂₁ oder H und R₃ R₃₁, -NR₅R₆ oder -OR₂,
oder X O, R₂ R₂₁ und R₃ R₃₁,
oder X O, R₂ H und R₃ -R₈OR₉, -R₈OCOR₉, -R₈NR₁₀R₁₁ oder -R₈R₁₂,
oder X und R₃ gemeinsam R₁₈;
- Y: O oder S;
- R₄: C₁-C₈-Alkyl, Halogen-C₁-C₈-Alkyl oder Halogen-C₁-C₈-Alkoxy;
- R₂₁: C₁-C₈-Alkyl, C₃-C₆-Cycloalkyl, Halogen-C₁-C₈-alkyl, C₁-C₄-Alkoxy-C₁-C₄-alkyl, C₁-C₄-Alkylthio-C₁-C₄-alkyl, R₁₃R₁₄N-C₁-C₄-alkyl, C₂-C₈-Alkenyl, C₂-C₈-Alkinyl, C₁-C₄-Acyl, CH=C(CN)₂ oder unsubstituiertes oder ein- bis fünffach substituiertes Phenyl, wobei die Substituenten ausgewählt sind aus der Gruppe, bestehend aus Halogen, C₁-C₄-Alkyl, C₂-C₅-Alkenyl, Halogen-C₁-C₄-alkyl, C₁-C₄-Alkoxy, SO₂NR₁₃R₁₄, Halogen-C₁-C₄-alkoxy, C₁-C₄-Alkylthio, C₁-C₄-Alkoxy-C₁-C₄-alkoxy, Halogen-C₁-C₄-alkylthio, NR₁₃R₁₄, -NO₂ und -CN;
- R₃₁: H, C₁-C₈-Alkyl, C₃-C₆-Cycloalkyl, C₃-C₆-Cycloalkyl-C₁-C₃-alkyl, Halogen-C₁-C₈-alkyl, Halogen-C₃-C₆-Cycloalkyl-C₁-C₃-alkyl, C₂-C₈-Alkenyl, C₂-C₈-Alkinyl, Halogen-C₂-C₈-Alkenyl, -COOR₇, -SR₂, -R₈OR₉, -R₈OCOR₉, -R₈NR₁₀R₁₁, -R₈R₁₂, -R₈SR₉, -R₈SCSR₉, unsubstituiertes oder ein- bis fünffach substituiertes Phenyl, wobei die Substituenten ausgewählt sind aus der Gruppe, bestehend aus Halogen, C₁-C₄-Alkyl, C₂-C₅-Alkenyl, Halogen-C₁-C₄-alkyl, Halogen-C₁-C₄-alkoxy, Halogen-C₁-C₄-alkylthio, SO₂NR₁₃R₁₄, C₁-C₄-Alkoxy, -NO₂ und -CN; ein unsubstituierter oder substituierter, gesättigter oder ungesättigter, fünf- oder sechsgliedriger, heterocyclischer oder heterobicyclischer Ring, enthaltend ein bis drei Heteroatome, ausgewählt aus der Gruppe O, N und S, wobei die Substituenten ausgewählt sind aus der Gruppe, bestehend aus Halogen, C₁-C₄-Alkyl, Halogen-C₁-C₄-Alkyl und C₁-C₄-Alkoxy;
- R₅ und R₆: unabhängig voneinander H, C₁-C₈-Alkyl, Halogen-C₁-C₈-Alkyl, C₂-C₅-Alkenyl, C₁-C₄-Alkoxy, C₂-C₅-Alkenoxy oder -NR₁₃R₁₄;
- R₇: H, C₁-C₈-Alkyl, Halogen-C₁-C₈-Alkyl oder C₂-C₈-Alkenyl;
- R₈: (CR₁₅R₁₆)ₚ, wobei p 1, 2, 3 oder 4 ist;
- R₉: C₁-C₈-Alkyl, Halogen-C₁-C₁₁-Alkyl, C₃-C₆-Cycloalkyl, C₂-C₈-Alkenyl, Halogen-C₂-C₁₁-Alkenyl, C₂-C₈-Alkinyl, NR₁₀R₁₁, OR₁₀, Benzyl oder unsubstituiertes oder ein- bis fünffach substituiertes Phenyl, wobei die Substituenten ausgewählt sind aus der Gruppe, bestehend aus Halogen, C₁-C₄-Alkyl, C₂-C₅-Alkenyl, Halogen-C₁-C₄-alkyl, C₁-C₄-Alkoxy, -NO₂ und -CN;
- R₁₀ und R₁₁: unabhängig voneinander C₁-C₈-Alkyl, C₂-C₈-Alkenyl oder C₁-C₈-Alkoxy-C₁-C₈-Alkyl;
- R₁₂: ein unsubstituierter oder substituierter, gesättigter oder ungesättigter, fünf- oder sechsgliedriger, heterocyclischer oder heterobicyclischer Ring, enthaltend ein bis drei Heteroatome, ausgewählt aus der Gruppe O, N und S, wobei die Substituenten ausgewählt sind aus der Gruppe, bestehend aus Halogen, C₁-C₄-Alkyl, Halogen-C₁-C₄-alkyl, C₁-C₄-Alkoxy;
- R₁₃ und R₁₄: unabhängig voneinander H oder C₁-C₈-Alkyl;
- R₁₅ und R₁₆: unabhängig voneinander H, C₁-C₈-Alkyl oder unsubstituiertes oder ein- bis fünffach substituiertes Phenyl, wobei die Substituenten ausgewählt sind aus der Gruppe, bestehend aus Halogen, C₁-C₄-Alkyl, C₂-C₅-Alkenyl, Halogen-C₁-C₄-alkyl, C₁-C₄-Alkoxy, -NO₂ und -CN;
- R₁₈: NC(R₁₉)=C(R₁₉)S, NC(R₁₉)=C(R₁₉)O, NC(R₁₉)₂C(R₁₉)₂S, NC(R₁₉)₂C(R₁₉)₂O, NC(R₁₉)=C(R₁₉)C(R₁₉)=N oder NC(R₁₉)=NC(R₁₉)=N;
- R₁₉: H, C₁-C₄-Alkyl, Halogen oder Halogen-C₁-C₄-alkyl;
- R₂₂: C₁-C₄-Alkyl, C₁-C₄-Alkoxy oder NR₁₃R₁₄; und
- R₂₃: C₁-C₄-Alkyl, C₁-C₄-Alkoxy, C₃-C₄-Alkylthio oder NR₁₃R₁₄
bedeuten,
mit der Massgabe, dass in den Verbindungen der Formel I R₂ von H verschieden ist, wenn entweder m 0, n 1, R₁ 4-Cl, R₃ -N(OCH₃)CH₃, R₄ CF₃ und X S ist, oder m 0, n 2, R₁ 3,4-Dichlor, R₃ -N(OCH₃)CH₃, R₄ CF₃ und X S ist, oder m 0, n 1, R₁ 4-Cl, R₃ -NHNH₂, R₄ CF₃ und X S ist, und gegebenenfalls Tautomere davon, jeweils in freier Form oder in Salzform;
ein Verfahren zur Herstellung und die Verwendung dieser Verbindungen und Tautomeren, Schädlingsbekämpfungsmittel, deren Wirkstoff aus diesen Verbindungen und Tautomeren, jeweils in freier Form oder in agrochemisch verwendbarer Salzform, ausgewählt ist, und ein Verfahren zur Herstellung und die Verwendung dieser Mittel.

In der Literatur (z. B. EP-A-3913 und EP-A-566 534) werden gewisse Hydrazonderivate als insektizid wirkende Wirkstoffe in Schädlingsbekämpfungsmitteln vorgeschlagen. Die biologischen Eigenschaften dieser bekannten Verbindungen vermögen auf dem Gebiet der Schädlingsbekämpfung jedoch nicht voll zu befriedigen, weshalb das Bedürfnis besteht, weitere Verbindungen mit schädlingsbekämpfenden Eigenschaften, insbesondere zur Bekämpfung von Insekten, Ektoparasiten und Vertretern der Ordnung Acarina, zur Verfügung zu stellen, wobei diese Aufgabe erfindungsgemäss durch die Bereitstellung der vorliegenden Verbindungen I gelöst wird.

Die Verbindungen I können teilweise als Tautomere vorliegen. Bedeutet z. B. R₂ H, so können entsprechende Verbindungen I, also solche mit einer -N(H)-C(R₃)=X-Teilstruktur, im Gleichgewicht mit den jeweiligen Tautomeren vorliegen, die eine -N=C(R₃)-XH-Teilstruktur aufweisen. Demgemäss sind unter den Verbindungen I nachstehend gegebenenfalls auch entsprechende Tautomere zu verstehen, auch wenn letztere nicht in jedem Fall speziell erwähnt werden.

Die Verbindungen I und gegebenenfalls ihre Tautomeren können als Salze vorliegen. Verbindungen I, welche mindestens ein basisches Zentrum aufweisen, können z. B. Säureadditionssalze bilden. Diese werden beispielsweise mit starken anorganischen Säuren, wie Mineralsäuren, z. B. Schwefelsäure, einer Phosphorsäure oder einer Halogenwasserstoffsäure, mit starken organischen Carbonsäuren, wie gegebenenfalls, z. B. durch Halogen, substituierten C₁-C₄-Alkancarbonsäuren, z. B. Essigsäure, wie gegebenenfalls ungesättigten Dicarbonsäuren, z. B. Oxal-, Malon-, Malein-, Fumar- oder Phthalsäure, wie Hydroxycarbonsäuren, z. B. Ascorbin-, Milch-, Äpfel-, Wein- oder Zitronensäure, oder wie Benzoesäure, oder mit organischen Sulfonsäuren, wie gegebenenfalls, z. B. durch Halogen, substituierten C₁-C₄-Alkan- oder Aryl-sulfonsäuren, z. B. Methan- oder p-Toluolsulfonsäure, gebildet. Ferner können Verbindungen I mit mindestens einer aciden Gruppe Salze mit Basen bilden. Geeignete Salze mit Basen sind beispielsweise Metallsalze, wie Alkali- oder Erdalkalimetallsalze, z. B. Natrium-, Kalium- oder Magnesiumsalze, oder Salze mit Ammoniak oder einem organischen Amin, wie Morpholin, Piperidin, Pyrrolidin, einem Mono-, Di- oder Triniederalkylamin, z. B. Ethyl-, Diethyl-, Triethyl- oder Dimethyl-propyl-amin, oder einem Mono-, Di- oder Trihydroxyniederalkylamin, z. B. Mono-, Di- oder Triethanolamin. Weiterhin können gegebenenfalls entsprechende innere Salze gebildet werden. Bevorzugt sind im Rahmen der Erfindung agrochemisch vorteilhafte Salze; umfasst sind aber auch für agrochemische Verwendungen mit Nachteilen behaftete, z. B. bienen- oder fisch-toxische, Salze, die beispielsweise für die Isolierung bzw. Reinigung von freien Verbindungen I oder deren agrochemisch verwendbaren Salzen eingesetzt werden. Infolge der engen Beziehung zwischen den Verbindungen I in freier Form und in Form ihrer Salze sind vorstehend und nachfolgend unter den freien Verbindungen I bzw. ihren Salzen sinn- und zweckgemäss gegebenenfalls auch die entsprechenden Salze bzw. die freien Verbindungen I zu verstehen. Entsprechendes gilt für Tautomere von Verbindungen I und deren Salze.

Die vor- und nachstehend verwendeten Allgemeinbegriffe haben, sofern nicht abweichend definiert, die nachfolgend aufgeführten Bedeutungen.

Halogen - als Gruppe per se sowie als Strukturelement von anderen Gruppen und Verbindungen, wie von Halogenalkyl, Halogencycloalkyl und Halogenalkenyl - ist Fluor, Chlor, Brom oder Iod, insbesondere Fluor, Chlor oder Brom, vor allem Fluor oder Chlor.

Kohlenstoffhaltige Gruppen und Verbindungen enthalten, sofern nicht abweichend definiert, jeweils 1 bis und mit 8, vorzugsweise 1 bis und mit 4, insbesondere 1 oder 2, Kohlenstoffatome.

C₃-C₆-Cycloalkyl ist Cyclopropyl, Cyclobutyl, Cyclopentyl oder Cyclohexyl.

Alkyl - als Gruppe per se sowie als Strukturelement von anderen Gruppen und Verbindungen, wie von Halogenalkyl, Alkoxy und Alkylthio, - ist, jeweils unter gebührender Berücksichtigung der von Fall zu Fall umfassten Anzahl der in der entsprechenden Gruppe oder Verbindung enthaltenen Kohlenstoffatome, entweder geradkettig, d. h. Methyl, Ethyl, Propyl, Butyl, Pentyl, Hexyl, Heptyl oder Octyl, oder verzweigt, z. B. Isopropyl, Isobutyl, sek.-Butyl, tert.-Butyl, Isopentyl, Neopentyl oder Isooctyl.

Halogen-substituierte kohlenstoffhaltige Gruppen und Verbindungen, wie Halogenalkyl, Halogencycloalkyl oder Halogenalkenyl, können teilweise halogeniert oder perhalogeniert sein, wobei im Falle von Mehrfach-Halogenierung die Halogensubstituenten gleich oder verschieden sein können. Beispiele für Halogenalkyl - als Gruppe per se sowie als Strukturelement von anderen Gruppen und Verbindungen, wie von Halogencycloalkylalkyl und Halogenalkenyl, - sind das ein- bis dreifach durch Fluor, Chlor und/oder Brom substituierte Methyl, wie CHF₂ oder CF₃; das ein- bis fünffach durch Fluor, Chlor und/oder Brom substituierte Ethyl, wie CH₂CF₃, CF₂CF₃, CF₂CCl₃, CF₂CHCl₂, CF₂CHF₂, CF₂CFCl₂, CF₂CHBr₂, CF₂CHClF, CF₂CHBrF oder CClFCHClF; das ein- bis siebenfach durch Fluor, Chlor und/oder Brom substituierte Propyl oder Isopropyl, wie CH₂CHBrCH₂Br, CF₂CHFCF₃, CH₂CF₂CF₃ oder CH(CF₃)₂; und das ein- bis neunfach durch Fluor, Chlor und/oder Brom substituierte Butyl oder eines seiner Isomeren, wie CF(CF₃)CHFCF₃ oder CH₂(CF₂)₂CF₃.

Acyl-Gruppen können Formyl, Acetyl, Propionyl, Butyryl oder Isobutyryl sein.

Beispiele für gesättigte heterocyclische Ringe sind Pyrrolidinyl, Piperidyl oder Morpholinyl, Beispiele für ungesättigte heterocyclische und heterobicyclische Ringe sind Thienyl, Pyrryl, Imidazolyl, Pyrazolyl, Triazolyl, Thiazolyl, Isothiazolyl, Pyridyl, Indolyl, Benzo[b]thienyl oder Benzo[b]furyl.

Bevorzugte Ausführungsformen im Rahmen der Erfindung, unter Berücksichtigung der vorstehend erwähnten Massgabe, sind:
(1) Eine Verbindung der Formel I, worin m 0, 1 oder 2, bevorzugt 0 oder 1, insbesondere 0 ist, wobei, wenn m grösser als 1 ist, die Reste R₁₇ gleich oder verschieden sind, oder gegebenenfalls ein Tautomeres davon;
(2) Eine Verbindung der Formel I, worin
   - n: 1, 2 oder 3,
   bevorzugt 1 oder 2,
   insbesondere 1 ist,
   wobei, wenn n grösser als 1 ist, die Reste R₁ gleich oder verschieden sind, oder gegebenenfalls ein Tautomeres davon;
(3) Eine Verbindung der Formel I, worin
   - R₁: Halogen, C₁-C₄-Alkyl oder Halogen-C₁-C₄-Alkyl,
   bevorzugt Halogen,
   insbesondere Fluor, Chlor oder Brom ist,
   oder gegebenenfalls ein Tautomeres davon;
(4) Eine Verbindung der Formel I, worin
   entweder X S, R₂ R₂₁ und R₃ R₃₁, -NR₅R₆ oder -OR₂,
   insbesondere X S, R₂ R₂₁ und R₃ R₃₁ ist;
   oder X O, R₂ R₂₁ und R₃ R₃₁ ist;
   oder X O, R₂ H und R₃ -R₈OR₉, -R₈OCOR₉, -R₈NR₁₀R₁₁ oder -R₈R₁₂,
   insbesondere X O, R₂ H und R₃ -R₈OR₉ ist;
(5) Eine Verbindung der Formel I, worin
   - R₂₁: C₁-C₄-Alkyl, C₃-C₆-Cycloalkyl, C₂-C₄-Alkenyl, C₂-C₄-Alkinyl, C₁-C₂-Acyl oder Phenyl,
   bevorzugt C₁-C₄-Alkyl, C₃-C₆-Cycloalkyl, C₁-C₄-Acyl oder Phenyl,
   insbesondere C₁-C₄-Alkyl, Formyl oder Acetyl ist,
   oder gegebenenfalls ein Tautomeres davon;
(6) Eine Verbindung der Formel I, worin
   - R₃₁: H, C₁-C₆-Alkyl, C₃-C₆-Cycloalkyl, C₃-C₆-Cycloalkylmethyl, Halogen-C₁-C₄-alkyl, Halogen-C₃-C₆-Cycloalkylmethyl, C₂-C₄-Alkenyl, C₂-C₄-Alkinyl, Halogen-C₂-C₄-Alkenyl, -COOR₇, -SR₂, -R₈OR₉, -R₈OCOR₉, -R₈NR₁₀R₁₁, -R₈R₁₂, -R₈SR₉, -R₈SCSR₉, Phenyl oder ein unsubstituierter oder substituierter, ungesättigter, fünf- oder sechsgliedriger, heterocyclischer oder heterobicyclischer Ring, enthaltend ein bis drei Heteroatome, ausgewählt aus der Gruppe O, N und S, wobei die Substituenten ausgewählt sind aus der Gruppe, bestehend aus Halogen, C₁-C₄-Alkyl, C₁-C₄-Alkoxy,
   bevorzugt H, C₁-C₆-Alkyl, C₃-C₆-Cycloalkyl, Halogen-C₁-C₃-alkyl, Halogen-C₃-C₆-Cycloalkylmethyl, C₂-C₄-Alkenyl, Halogen, -COOR₇, -SR₂, -R₈OR₉, -R₈OCOR₉, -R₈NR₁₀R₁₁, -R₈R₁₂, Phenyl oder ein unsubstituierter oder substituierter, ungesättigter, fünf- oder sechsgliedriger, heterocyclischer oder heterobicyclischer Ring, enthaltend ein bis drei Heteroatome, ausgewählt aus der Gruppe O, N und S, wobei die Substituenten ausgewählt sind aus der Gruppe Halogen;
   insbesondere H, C₁-C₄-Alkyl, C₃-C₆-Cycloalkyl, Halogen-C₁-C₂-alkyl, Halogen-Cyclopropylmethyl, C₂-C₄-Alkenyl, Fluor, Chlor, -SR₂, -R₈OCOR₉, -R₈R₁₂ oder ein unsubstituierter oder substituierter, ungesättigter, fünf- oder sechsgliedriger, heterocyclischer oder heterobicyclischer Ring, enthaltend N oder S, wobei die Substituenten ausgewählt sind aus der Gruppe Fluor und Chlor,
   oder gegebenenfalls ein Tautomeres davon;
(7) Eine Verbindung der Formel I, worin
   - R₄: C₁-C₄-Alkyl oder Halogen-C₁-C₄-Alkyl,
   bevorzugt Halogen-C₁-C₄-Alkyl,
   insbesondere Halogen-C₁-C₂-Alkyl,
   ganz besonders Trifluormethyl ist,
   oder gegebenenfalls ein Tautomeres davon;
(8) Eine Verbindung der Formel I, worin
   - R₅ und R₆: unabhängig voneinander H, C₁-C₄-Alkyl, Halogen-C₁-C₄-Alkyl, C₂-C₄-Alkenyl, C₁-C₃-Alkoxy, C₂-C₃-Alkenoxy oder -NR₁₃R₁₄,
   bevorzugt H, C₁-C₂-Alkyl, Halogen-C₁-C₂-Alkyl, C₂-C₃-Alkenyl, C₁-C₂-Alkoxy, C₂-C₃-Alkenoxy oder -NR₁₃R₁₄,
   insbesondere H, C₁-C₂-Alkyl, C₂-C₃-Alkenyl, C₁-C₂-Alkoxy, C₂-C₃-Alkenoxy oder -NR₁₃R₁₄ sind,
   oder gegebenenfalls ein Tautomeres davon;
(9) Eine Verbindung der Formel I, worin
   - R₇: C₁-C₄-Alkyl, Halogen-C₁-C₄-Alkyl oder C₂-C₄-Alkenyl,
   bevorzugt C₁-C₂-Alkyl oder Halogen-C₁-C₂-Alkyl,
   insbesondere Methyl oder Ethyl ist,
   oder gegebenenfalls ein Tautomeres davon;
(10) Eine Verbindung der Formel I, worin
   - R₈: (CR₁₅R₁₆)ₚ, wobei p 1, 2 oder 3,
   bevorzugt 1 oder 2,
   insbesondere 1 ist,
   oder gegebenenfalls ein Tautomeres davon;
(11) Eine Verbindung der Formel I, worin
   - R₉: C₁-C₄-Alkyl, Halogen-C₁-C₁₁-Alkyl, C₃-C₆-Cycloalkyl, C₂-C₄-Alkenyl, Halogen-C₂-C₁₁-Alkenyl, C₂-C₄-Alkinyl, NR₁₀R₁₁, OR₁₀, Benzyl oder unsubstituiertes oder einfach substituiertes Phenyl, wobei die Substituenten ausgewählt sind aus der Gruppe, bestehend aus Halogen, C₁-C₂-Alkyl, Halogen-C₁-C₂-alkyl, C₁-C₃-Alkoxy, -NO₂ und -CN;
   bevorzugt C₁-C₄-Alkyl, Halogen-C₈-C₁₁-Alkyl, C₃-C₅-Cycloalkyl, C₂-C₃-Alkenyl, Halogen-C₈-C₁₁-Alkenyl, C₂-C₃-Alkinyl oder unsubstituiertes oder einfach substituiertes Phenyl, wobei die Substituenten ausgewählt sind aus der Gruppe, bestehend aus Fluor, Chlor, Methyl, C₁-C₂-Alkoxy, und -NO₂;
   insbesondere C₁-C₄-Alkyl, Halogen-C₈-C₁₁-Alkyl, Cyclopropyl, Vinyl, Halogen-C₈-C₁₁-Alkenyl, Ethinyl oder unsubstituiertes oder einfach substituiertes Phenyl, wobei die Substituenten ausgewählt sind aus der Gruppe, bestehend aus Chlor, Methyl, Methoxy, und -NO₂; ist,
   oder gegebenenfalls ein Tautomeres davon;
(12) Eine Verbindung der Formel I, worin
   - R₁₀ und R₁₁: unabhängig voneinander C₁-C₄-Alkyl, C₂-C₄-Alkenyl oder C₁-C₄-Alkoxy-C₁-C₄-Alkyl,
   bevorzugt C₁-C₂-Alkyl, C₂-C₃-Alkenyl oder C₁-C₂-Alkoxy-C₁-C₂-Alkyl,
   insbesondere Methyl, Allyl oder Methoxyethyl ist,
   oder gegebenenfalls ein Tautomeres davon;
(13) Eine Verbindung der Formel I, worin
   - R₁₂: ein unsubstituierter oder substituierter, ungesättigter, fünf- oder sechsgliedriger, heterocyclischer oder heterobicyclischer Ring, enthaltend ein bis drei Heteroatome, ausgewählt aus der Gruppe O, N und S, wobei die Substituenten ausgewählt sind aus der Gruppe, bestehend aus Halogen, C₁-C₄-Alkyl, C₁-C₄-Alkoxy;
   bevorzugt ein unsubstituierter, ungesättigter, fünf- oder sechsgliedriger, heterocyclischer oder heterobicyclischer Ring, enthaltend ein bis drei Heteroatome, ausgewählt aus der Gruppe O, N und S,
   insbesondere ein unsubstituierter, ungesättigter, fünfgliedriger, heterocyclischer Ring, enthaltend ein bis drei Heteroatome, ausgewählt aus der Gruppe N und S,
   ganz besonders Imidazolyl, Pyrazolyl oder Triazolyl ist,
   oder gegebenenfalls ein Tautomeres davon;
(14) Eine Verbindung der Formel I, worin
   - R₁₃ und R₁₄: unabhängig voneinander H oder C₁-C₄-Alkyl, bevorzugt H oder C₁-C₂-Alkyl,
   insbesondere H oder Methyl sind, oder gegebenenfalls ein Tautomeres davon;
(15) Eine Verbindung der Formel I, worin
   - R₁₅ und R₁₆: unabhängig voneinander H, C₁-C₄-Alkyl oder unsubstituiertes oder einfach substituiertes Phenyl, wobei die Substituenten ausgewählt sind aus der Gruppe, bestehend aus Halogen, C₁-C₂-Alkyl, C₂-C₃-Alkenyl, Halogen-C₁-C₃-alkyl, C₁-C₂-Alkoxy, -NO₂ und -CN;
   bevorzugt H, C₁-C₂-Alkyl oder Phenyl,
   insbesondere H, Methyl oder Phenyl sind,
   oder gegebenenfalls ein Tautomeres davon;
(16) Eine Verbindung der Formel I, worin
   - R₁₇: Halogen oder C₁-C₄-Alkyl,
   bevorzugt Chlor oder Methyl,
   oder gegebenenfalls ein Tautomeres davon;
(17) Eine Verbindung der Formel I, worin
   - R₁₈: NC(R₁₉)=C(R₁₉)S, NC(R₁₉)=C(R₁₉)O, NC(R₁₉)=C(R₁₉)C(R₁₉)=N oder N(R₁₉)=NC(R₁₉)=N,
   bevorzugt NC(R₁₉)=C(R₁₉)S, NC(R₁₉)=C(R₁₉)O oder NC(R₁₉)=C(R₁₉)C(R₁₉)=N,
   insbesondere NC(R₁₉)=C(R₁₉)S oder NC(R₁₉)=C(R₁₉)O,
   oder gegebenenfalls ein Tautomeres davon;
(18) Eine Verbindung der Formel I, worin
   - m: 0,
   - n: 1,
   - R₁: Fluor, Chlor oder Brom,
   - R₂: C₁-C₄-Alkyl, C₃-C₆-Cycloalkyl, C₁-C₄-Acyl oder Phenyl,
   - R₃: H, C₁-C₆-Alkyl, C₃-C₆-Cycloalkyl, Halogen-C₁-C₃-alkyl, Halogen-C₃-C₆-Cycloalkylmethyl, C₂-C₄-Alkenyl, -COOR₇, -SR₂, -R₈OR₉, -R₈OCOR₉ -R₈NR₁₀R₁₁, -R₈R₁₂, Phenyl oder ein unsubstituierter oder substituierter, ungesättigter, fünf- oder sechsgliedriger, heterocyclischer oder heterobicyclischer Ring, enthaltend ein bis drei Heteroatome, ausgewählt aus der Gruppe O, N und S, wobei die Substituenten ausgewählt sind aus der Gruppe Halogen,
   - R₄: C₁-C₄-Alkyl oder Halogen-C₁-C₄-Alkyl,
   - R₇: C₁-C₂-Alkyl oder Halogen-C₁-C₂-Alkyl,
   - R₈: CR₁₅R₁₆,
   - R₉: C₁-C₄-Alkyl, Halogen-C₈-C₁₁-Alkyl, C₃-C₅-Cycloalkyl, C₂-C₃-Alkenyl, Halogen-C₈-C₁₁-Alkenyl, C₂-C₃-Alkinyl oder unsubstituiertes oder einfach substituiertes Phenyl, wobei die Substituenten ausgewählt sind aus der Gruppe, bestehend aus Fluor, Chlor, Methyl, C₁-C₂-Alkoxy, und -NO₂;
   - R₁₀ und R₁₁: unabhängig voneinander Methyl, Allyl oder Methoxyethyl, R₁₂ ein unsubstituierter, ungesättigter, fünfgliedriger, heterocyclischer Ring, enthaltend ein bis drei Heteroatome, ausgewählt aus der Gruppe N und S, und
   - R₁₅ und R₁₆: unabhängig voneinander H, Methyl oder Phenyl ist,
   oder gegebenenfalls ein Tautomeres davon;
(19) Eine Verbindung der Formel I, worin
   - m: 0,
   - n: 1,
   - R₁: Fluor, Chlor oder Brom,
   - R₂: C₁-C₄-Alkyl, Formyl oder Acetyl,
   - R₃: H, C₁-C₆-Alkyl, C₃-C₆-Cycloalkyl, Halogen-C₁-C₃-alkyl, Halogen-C₃-C₆-Cycloalkylmethyl, C₂-C₄-Alkenyl, -COOR₇, -SR₂, -R₈OR₉, -R₈OCOR₉ -R₈NR₁₀R₁₁, -R₈R₁₂, Phenyl oder ein unsubstituierter oder substituierter, ungesättigter, fünf- oder sechsgliedriger, heterocyclischer oder heterobicyclischer Ring, enthaltend ein bis drei Heteroatome, ausgewählt aus der Gruppe O, N und S, wobei die Substituenten ausgewählt sind aus der Gruppe Halogen,
   - R₄: Halogen-C₁-C₂-Alkyl,
   - R₇: Methyl oder Ethyl,
   - R₈: CR₁₅R₁₆,
   - R₉: C₁-C₄-Alkyl, Halogen-C₈-C₁₁-Alkyl, Cyclopropyl, Vinyl, Halogen-C₈-C₁₁-Alkenyl, Ethinyl oder unsubstituiertes oder einfach substituiertes Phenyl, wobei die Substituenten ausgewählt sind aus der Gruppe, bestehend aus Chlor, Methyl, Methoxy, und -NO₂;
   - R₁₀ und R₁₁: unabhängig voneinander Methyl, Allyl oder Methoxyethyl,
   - R₁₂: ein unsubstituierter, ungesättigter, fünfgliedriger, heterocyclischer Ring, enthaltend ein bis drei Heteroatome, ausgewählt aus der Gruppe N und S, und
   - R₁₅ und R₁₆: unabhängig voneinander H, Methyl oder Phenyl ist, oder gegebenenfalls ein Tautomeres davon;
(20) Eine Verbindung der Formel I, worin
   - m: 0,
   - n: 1,
   - R₁: Fluor, Chlor oder Brom,
   - R₂: C₁-C₄-Alkyl, Formyl oder Acetyl,
   - R₃: H, C₁-C₄-Alkyl, C₃-C₆-Cycloalkyl, Halogen-C₁-C₂-alkyl, Halogen-Cyclopropylmethyl, C₂-C₄-Alkenyl, -COOR₇, -SR₂, -R₈-OCOR₉, -R₈-R₁₂ oder ein unsubstituierter oder substituierter, ungesättigter, fünf- oder sechsgliedriger, heterocyclischer oder heterobicyclischer Ring, enthaltend N oder S, wobei die Substituenten ausgewählt sind aus der Gruppe Fluor und Chlor;
   - R₄: Trifluormethyl,
   - R₇: Methyl oder Ethyl,
   - R₈: CR₁₅R₁₆,
   - R₉: C₁-C₄-Alkyl, Halogen-C₈-C₁₁-Alkyl, Cyclopropyl, Vinyl, Halogen-C₈-C₁₁-Alkenyl, Ethinyl oder unsubstituiertes oder einfach substituiertes Phenyl, wobei die Substituenten ausgewählt sind aus der Gruppe, bestehend aus Chlor, Methyl, Methoxy, und -NO₂;
   - R₁₂: ein unsubstituierter, ungesättigter, fünfgliedriger, heterocyclischer Ring, enthaltend ein bis drei Heteroatome, ausgewählt aus der Gruppe N und S, und
   - R₁₅ und R₁₆: unabhängig voneinander N, Methyl oder Phenyl ist, oder gegebenenfalls ein Tautomeres davon;
(21) Eine Verbindung der Formel I, worin
   - m: 0,
   - n: 1,
   - R₁: Fluor, Chlor oder Brom,
   - R₂: C₁-C₄-Alkyl, Formyl oder Acetyl,
   - R₃: H, C₁-C₄-Alkyl, C₃-C₆-Cycloalkyl, Halogen-C₁-C₂-alkyl, Halogen-Cyclopropylmethyl, C₂-C₄-Alkenyl, -COOR₇, -SR₂, -R₈-OCOR₉, -R₈-R₁₂ oder ein unsubstituierter oder substituierter, ungesättigter, fünf- oder sechsgliedriger, heterocyclischer oder heterobicyclischer Ring, enthaltend N oder S, wobei die Substituenten ausgewählt sind aus der Gruppe Fluor und Chlor;
   - R₄: Trifluormethyl,
   - R₇: Methyl oder Ethyl,
   - R₈: CH₂, C(Methyl)₂ oder CH(Phenyl),
   - R₉: C₁-C₄-Alkyl, Halogen-C₈-C₁₁-Alkyl, Cyclopropyl, Vinyl, Halogen-C₈-C₁₁-Alkenyl, Ethinyl oder unsubstituiertes oder einfach substituiertes Phenyl, wobei die Substituenten ausgewählt sind aus der Gruppe, bestehend aus Chlor, Methyl, Methoxy, und -NO₂; und
   - R₁₂: ein unsubstituierter, ungesättigter, fünfgliedriger, heterocyclischer Ring, enthaltend ein bis drei Heteroatome, ausgewählt aus der Gruppe N und S,
   oder gegebenenfalls ein Tautomeres davon.

Besonders bevorzugt sind im Rahmen der Erfindung die in Tabelle 1 aufgeführten und ganz besonders bevorzugt die in den Beispielen H2, H3, H5 bis H13, H15, H16 und H18 genannten Verbindungen der Formel I und gegebenenfalls deren Tautomere.

Namentlich bevorzugt ist im Rahmen der Erfindung 4-Chlor-4'-trifluormethansulfonyloxybenzophenon-N-formyl-N-methylhydrazon (Verbindung 1 in Tabelle 1).

Als weiterer Gegenstand der Erfindung ist das Verfahren zur Herstellung der Verbindungen der Formel I und gegebenenfalls ihrer Tautomeren, unter Berücksichtigung der vorstehend erwähnten Massgabe, jeweils in freier Form oder in Salzform, z. B. dadurch gekennzeichnet, dass man
a) eine Verbindung der Formel 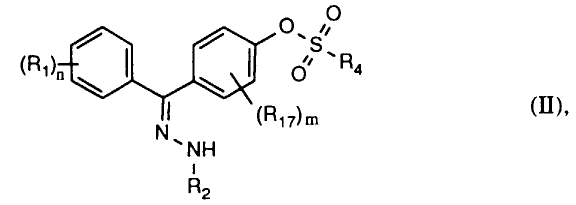 die bekannt ist oder in Analogie zu entsprechenden bekannten Verbindungen hergestellt werden kann und worin m, n, R₁, R₂, R₄ und R₁₇ die für die Formel I angegebenen Bedeutungen haben, oder gegebenenfalls ein Tautomeres davon, in freier Form oder in Salzform, vorzugsweise in Gegenwart einer Base, mit Phosgen oder Thiophosgen umsetzt und das gegebenenfalls isolierte Zwischenprodukt, eventuell in Gegenwart einer Base, mit einer Verbindung der Formel Y₁H, worin Y₁ -NR₅R₆, -OR₂ oder -SR₂ ist und R₂, R₅ und R₆ die für die Formel I angegebenen Bedeutungen haben, oder einem Salz davon, umsetzt oder
b) zur Herstellung einer Verbindung der Formel I, worin X O ist, eine Verbindung der Formel II, eventuell in Gegenwart einer Base, mit einer Verbindung der Formel R₃COY₂, worin R₃ die für die Formel I angegebenen Bedeutungen mit Ausnahme von -OR₂ und -SR₂ hat und Y₂ eine Abgangsgruppe ist, umsetzt oder
c) zur Herstellung einer Verbindung der Formel I, worin X S ist, eine Verbindung der Formel II mit einer Verbindung der Formel Y₃-N=C=S, worin Y₃ H, C₁-C₈-Alkyl, Halogen-C₁-C₈-Alkyl oder C₂-C₅-Alkenyl, bevorzugt H oder C₁-C₈-Alkyl, bedeutet, zu Verbindungen der Formel I, worin X S ist, umsetzt oder
d) zur Herstellung einer Verbindung der Formel I, worin X S und R₃ -SR, worin R C₁-C₈-Alkyl, C₃-C₆-Cycloalkyl, C₂-C₈-Alkenyl oder C₂-C₈-Alkinyl, bevorzugt C₁-C₈-Alkyl, C₃-C₆-Cycloalkyl, insbesondere C₁-C₈-Alkyl bedeutet, ist, eine Verbindung der Formel II, eventuell in Gegenwart einer Base, mit CS₂ umsetzt und das gegebenenfalls isolierte Zwischenprodukt, eventuell in Gegenwart einer Base, mit einer Verbindung der Formel RY₄, worin R die oben definierten Bedeutungen hat und Y₄ eine Abgangsgruppe ist, umsetzt oder
e) zur Herstellung einer Verbindung der Formel I, worin X O ist, eine Verbindung der Formel II, eventuell in Gegenwart einer Base, mit einer Verbindung der Formel ClR₈COY₅, worin R₈ die für die Formel I angegebenen Bedeutungen hat und Y₅ eine Abgangsgruppe ist, umsetzt und das entstandene Zwischenprodukt, eventuell in Gegenwart einer Base, eventuell in Gegenwart eines Alkalimetallhalogenids, wie in Gegenwart von Natriumiodid, mit einer Verbindung der Formel R₉COOH, HNR₁₀R₁₁ oder R₁₂, worin R₉, R₁₀, R₁₁ und R₁₂ die für die Formel I angegebenen Bedeutungen haben, umsetzt oder
f) zur Herstellung einer Verbindung der Formel I, worin X S ist, eine Verbindung der Formel 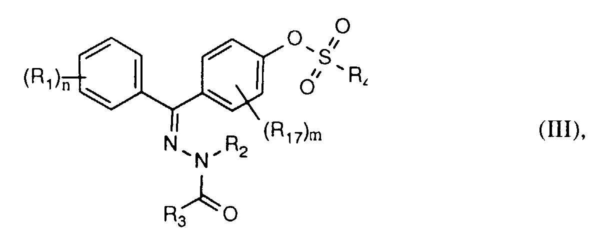 die bekannt ist oder in Analogie zu entsprechenden bekannten Verbindungen, z. B. nach der Variante b), hergestellt werden kann und worin m, n, R₁, R₂, R₃, R₄ und R₁₇ die für die Formel I angegebenen Bedeutungen haben, mit P₂S₅ umsetzt, oder
g) zur Herstellung einer Verbindung der Formel I, worin X S und R₂ H ist, eine Verbindung der Formel 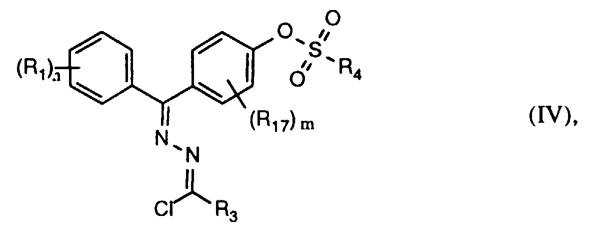 die bekannt ist oder in Analogie zu entsprechenden bekannten Verbindungen hergestellt werden kann und worin m, n, R₁, R₃, R₄ und R₁₇ die für die Formel I angegebenen Bedeutungen haben, eventuell in Gegenwart einer Base, mit H₂S umsetzt oder
h) eine Verbindung der Formel I, worin R₂ H ist, eventuell in Gegenwart einer Base, mit einer Verbindung der Formel R₂Y₆, worin R₂ die für die Formel I angegebenen Bedeutungen mit Ausnähme von H hat und Y₆ eine Abgangsgruppe ist, umsetzt oder
i) eine Verbindung der Formel 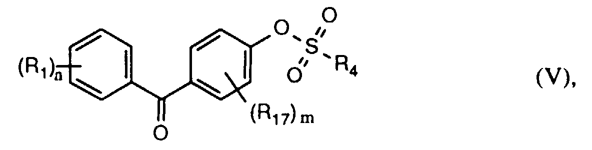 die bekannt ist oder in Analogie zu entsprechenden bekannten Verbindungen hergestellt werden kann und worin m, n, R₁, R₄ und R₁₇ die für die Formel I angegebenen Bedeutungen haben, eventuell in Gegenwart eines Säurekatalysators, eventuell in Gegenwart eines wasserbindenden Mittels, mit einer Verbindung der Formel 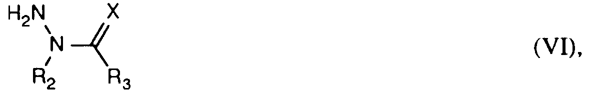 die bekannt ist oder in Analogie zu entsprechenden bekannten Verbindungen hergestellt werden kann und worin R₂, R₃ und X die für die Formel I angegebenen Bedeutungen haben, oder einem Salz und/oder gegebenenfalls einem Tautomeren davon umsetzt und jeweils, wenn erwünscht, eine verfahrensgemäss oder auf andere Weise erhältliche Verbindung der Formel I oder ein Tautomeres davon, jeweils in freier Form oder in Salzform, in eine andere Verbindung der Formel I oder ein Tautomeres davon überführt, ein verfahrensgemäss erhältliches Gemisch von Isomeren auftrennt und das gewünschte Isomere isoliert und/oder eine verfahrensgemäss erhältliche freie Verbindung der Formel I oder ein Tautomeres davon in ein Salz oder ein verfahrensgemäss erhältliches Salz einer Verbindung der Formel I oder eines Tautomeren davon in die freie Verbindung der Formel I oder ein Tautomeres davon oder in ein anderes Salz überführt.

Für vor- und nachstehend aufgeführte Ausgangsmaterialien gilt im Hinblick auf deren Tautomere bzw. Salze das vorstehend für Tautomere bzw. Salze von Verbindungen I Gesagte in analoger Weise.

Die vor- und nachstehend beschriebenen Umsetzungen werden in an sich bekannter Weise durchgeführt, z. B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z. B. in einem Temperaturbereich von etwa -80°C bis zur Siedetemperatur des Reaktionsmediums, vorzugsweise von etwa -20°C bis etwa +150°C, und, falls erforderlich, in einem geschlossenen Gefäss, unter Druck, in einer Inertgasatmosphäre und/oder unter wasserfreien Bedingungen arbeitet. Besonders vorteilhafte Reaktionsbedingungen können den Beispielen entnommen werden.

Die vor- und nachstehend aufgeführten Ausgangsmaterialien, die für die Herstellung der Verbindungen I und gegebenenfalls ihrer Tautomeren, jeweils in freier Form oder in Salzform, verwendet werden, sind bekannt oder können nach an sich bekannten Methoden, z. B. gemäss den nachstehenden Angaben, hergestellt werden.

### Variante a):

Geeignete Basen zur Erleichterung der Umsetzung sind z. B. Alkylamine, Alkylendiamine, gegebenenfalls N-alkylierte, gegebenenfalls ungesättigte, Cycloalkylamine sowie basische Heterocyclen. Beispielhaft seien Triethylamin, Diisopropyl-ethyl-amin, Triethylendiamin, N-Cyclohexyl-N,N-dimethyl-amin, N,N-Diethylanilin, Pyridin, 4-(N,N-Dimethylamino)pyridin, Chinuclidin, N-Methylmorpholin wie 1,5-Diazabicyclo[5.4.0]undec-5-en (DBU) genannt.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Mesitylen, Tetralin, Chlorbenzol, Dichlorbenzol, Brombenzol, Petrolether, Hexan, Cyclohexan, Dichlormethan, Trichlormethan, Tetrachlormethan, Dichlorethan, Trichlorethen oder Tetrachlorethen; Ester, wie Essigsäureethylester; Ether, wie Diethylether, Dipropylether, Diisopropylether, Dibutylether, tert.-Butylmethylether, Ethylenglykolmonomethylether, Ethylenglykolmonoethylether, Ethylenglykoldimethylether, Dimethoxydiethylether, Tetrahydrofuran oder Dioxan; Ketone, wie Aceton, Methylethylketon oder Methylisobutylketon; Amide, wie N,N-Dimethylformamid, N,N-Diethylformamid, N,N-Dimethylacetamid, N-Methylpyrrolidon oder Hexamethylphosphorsäuretriamid; Nitrile, wie Acetonitril oder Propionitril; und Sulfoxide, wie Dimethylsulfoxid. Wird die Umsetzung in Gegenwart einer Base ausgeführt, können auch im Ueberschuss eingesetzte Basen, wie Triethylamin, Pyridin, N-Methylmorpholin oder N,N-Diethylanilin, als Lösungs- oder Verdünnungsmittel dienen.

Die Umsetzung erfolgt vorteilhaft in einem Temperaturbereich von etwa -30°C bis etwa +100°C, bevorzugt von etwa -20°C bis etwa +20°C.

In einer bevorzugten Ausführungsform der Variante a) wird eine Verbindung II bei -20° bis 0°, vorzugsweise -10°, in einem aromatischen Kohlenwasserstoff, vorzugsweise in Toluol, und in Gegenwart einer Sulfonsäre als Katalysator, vorzugsweise in Gegenwart von Triethylamin mit Phosgen umgesetzt, das Produkt isoliert und mit Y₁H weiter umgesetzt.

### Variante b):

Geeignete Abgangsgruppen sind z. B. Halogene, C₁-C₈-Alkoxy oder C₁-C₈-Alkylcarboxy, vorzugsweise Halogene, insbesondere Chlor.

Geeignete Basen zur Erleichterung der Umsetzung sind z. B. Alkylamine, Alkylendiamine, gegebenenfalls N-alkylierte, gegebenenfalls ungesättigte, Cycloalkylamine sowie basische Heterocyclen. Beispielhaft seien Triethylamin, Diisopropyl-ethyl-amin, Triethylendiamin, N-Cyclohexyl-N,N-dimethyl-amin, N,N-Diethylanilin, Pyridin, 4-(N,N-Dimethylamino)pyridin, Chinuclidin, N-Methylmorpholin wie 1,5-Diazabicyclo[5.4.0]undec-5-en (DBU) genannt.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Mesitylen, Tetralin, Chlorbenzol, Dichlorbenzol, Brombenzol, Petrolether, Hexan, Cyclohexan, Dichlormethan, Trichlormethan, Tetrachlormethan, Dichlorethan, Trichlorethen oder Tetrachlorethen; Ester, wie Essigsäureethylester; Ether, wie Diethylether, Dipropylether, Diisopropylether, Dibutylether, tert.-Butylmethylether, Ethylenglykolmonomethylether, Ethylenglykolmonoethylether, Ethylenglykoldimethylether, Dimethoxydiethylether, Tetrahydrofuran oder Dioxan; Ketone, wie Aceton, Methylethylketon oder Methylisobutylketon; Amide, wie N,N-Dimethylformamid, N,N-Diethylformamid, N,N-Dimethylacetamid, N-Methylpyrrolidon oder Hexamethylphosphorsäuretriamid; Nitrile, wie Acetonitril oder Propionitril; und Sulfoxide, wie Dimethylsulfoxid. Wird die Umsetzung in Gegenwart einer Base ausgeführt, können auch im Ueberschuss eingesetzte Basen, wie Triethylamin, Pyridin, N-Methylmorpholin oder N,N-Diethylanilin, als Lösungs- oder Verdünnungsmittel dienen.

Die Umsetzung erfolgt vorteilhaft in einem Temperaturbereich von etwa -30°C bis etwa +100°C, bevorzugt von etwa -10°C bis etwa +20°C.

In einer bevorzugten Ausführungsform der Variante b) wird eine Verbindung II bei -20° bis 0°, vorzugsweise 0°, in einem Ether, vorzugsweise in Diethylether mit einem der genannten Acylierungsmittel umgesetzt.

### Variante c):

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Mesitylen, Tetralin, Chlorbenzol, Dichlorbenzol, Brombenzol, Petrolether, Hexan, Cyclohexan, Dichlormethan, Trichlormethan, Tetrachlormethan, Dichlorethan, Trichlorethen oder Tetrachlorethen; Ester, wie Essigsäureethylester; Ether, wie Diethylether, Dipropylether, Diisopropylether, Dibutylether, tert.-Butylmethylether, Ethylenglykolmonomethylether, Ethylenglykolmonoethylether, Ethylenglykoldimethylether, Dimethoxydiethylether, Tetrahydrofuran oder Dioxan; Ketone, wie Aceton, Methylethylketon oder Methylisobutylketon; Alkohole, wie Methanol, Ethanol, Propanol, Isopropanol, Butanol, Ethylenglykol oder Glycerin; Amide, wie N,N-Dimethylformamid, N,N-Diethylformamid, N,N-Dimethylacetamid, N-Methylpyrrolidon oder Hexamethylphosphorsäuretriamid; Nitrile, wie Acetonitril oder Propionitril; und Sulfoxide, wie Dimethylsulfoxid.

Die Umsetzung erfolgt vorteilhaft in einem Temperaturbereich von etwa -30°C bis etwa +100°C, bevorzugt von etwa -10°C bis etwa +20°C.

### Variante d):

Geeignete Abgangsgruppen sind z. B. Halogene oder C₁-C₈-Alkylsulfate, vorzugsweise Halogene, insbesondere Brom oder Iod, ganz besonders Iod.

Geeignete Basen zur Erleichterung der Umsetzung sind z. B. Alkalimetall- oder Erdalkalimetallhydroxide, -hydride, -amide, -alkanolate, -acetate, -carbonate, -dialkylamide oder -alkylsilylamide, Alkylamine, Alkylendiamine, gegebenenfalls N-alkylierte, gegebenenfalls ungesättigte, Cycloalkylamine, basische Heterocyclen, Ammoniumhydroxide sowie carbocyclische Amine. Beispielhaft seien Natriumhydroxid, -hydrid, -amid, -methanolat, -acetat, -carbonat, Kalium-tert.-butanolat, -hydroxid, -carbonat, -hydrid, Lithiumdiisopropylamid, Kalium-bis(trimethylsilyl)amid, Calciumhydrid, Triethylamin, Diisopropylethylamin, Triethylendiamin, Cyclohexylamin, N-Cyclohexyl-N,N-dimethylamin, N,N-Diethylanilin, Pyridin, 4-(N,N-Dimethylamino)pyridin, Chinuclidin, N-Methylmorpholin, Benzyltrimethylammoniumhydroxid sowie 1,5-Diazabicyclo[5.4.0]undec-5-en (DBU) genannt. Besonders geeignet sind Alkalimetall- oder Erdalkalimetallhydride, ganz besonders Alkalimetallhydride.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Mesitylen, Tetralin, Chlorbenzol, Dichlorbenzol, Brombenzol, Petrolether, Hexan, Cyclohexan, Dichlormethan, Trichlormethan, Tetrachlormethan, Dichlorethan, Trichlorethen oder Tetrachlorethen; Ester, wie Essigsäureethylester; Ether, wie Diethylether, Dipropylether, Diisopropylether, Dibutylether, tert.-Butylmethylether, Ethylenglykolmonomethylether, Ethylenglykolmonoethylether, Ethylenglykoldimethylether, Dimethoxydiethylether, Tetrahydrofuran oder Dioxan; Ketone, wie Aceton, Methylethylketon oder Methylisobutylketon; Amide, wie N,N-Dimethylformamid, N,N-Diethylformamid, N,N-Dimethylacetamid, N-Methylpyrrolidon oder Hexamethylphosphorsäuretriamid; Nitrile, wie Acetonitril oder Propionitril; und Sulfoxide, wie Dimethylsulfoxid. Wird die Umsetzung in Gegenwart einer Base ausgeführt, können auch im Ueberschuss eingesetzte Basen, wie Triethylamin, Pyridin, N-Methylmorpholin oder N,N-Diethylanilin, als Lösungs- oder Verdünnungsmittel dienen.

Die Umsetzung erfolgt vorteilhaft in einem Temperaturbereich von etwa -30°C bis etwa +100°C, bevorzugt von etwa -10°C bis etwa +20°C.

In einer bevorzugten Ausführungsform der Variante d) wird eine Verbindung II bei -20° bis 0°, vorzugsweise 0°, in Gegenwart eines Alkalimetallhydrids, vorzugsweise Natriumhydrid, in einem Carbonsäureamid, vorzugsweise in Dimethylformamid, mit CS₂ und danach ohne Isolation des Zwischenproduktes mit einem der genannten Alkylierungsmittel umgesetzt.

### Variante e):

Geeignete Abgangsgruppen sind z. B. Halogene oder C₁-C₈-Alkoxy, vorzugsweise Halogene, insbesondere Chlor.

Geeignete Basen zur Erleichterung der Umsetzung sind z. B. Alkylamine, Alkylendiamine, gegebenenfalls N-alkylierte, gegebenenfalls ungesättigte, Cycloalkylamine sowie basische Heterocyclen. Beispielhaft seien Triethylamin, Diisopropyl-ethyl-amin, Triethylendiamin, N-Cyclohexyl-N,N-dimethyl-amin, N,N-Diethylanilin, Pyridin, 4-(N,N-Dimethylamino)pyridin, Chinuclidin, N-Methylmorpholin wie 1,5-Diazabicyclo[5.4.0]undec-5-en (DBU) genannt.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Mesitylen, Tetralin, Chlorbenzol, Dichlorbenzol, Brombenzol, Petrolether, Hexan, Cyclohexan, Dichlormethan, Trichlormethan, Tetrachlormethan, Dichlorethan, Trichlorethen oder Tetrachlorethen; Ester, wie Essigsäureethylester; Ether, wie Diethylether, Dipropylether, Diisopropylether, Dibutylether, tert.-Butylmethylether, Ethylenglykolmonomethylether, Ethylenglykolmonoethylether, Ethylenglykoldimethylether, Dimethoxydiethylether, Tetrahydrofuran oder Dioxan; Ketone, wie Aceton, Methylethylketon oder Methylisobutylketon; Amide, wie N,N-Dimethylformamid, N,N-Diethylformamid, N,N-Dimethylacetamid, N-Methylpyrrolidon oder Hexamethylphosphorsäuretriamid; Nitrile, wie Acetonitril oder Propionitril; und Sulfoxide, wie Dimethylsulfoxid. Wird die Umsetzung in Gegenwart einer Base ausgeführt, können auch im Ueberschuss eingesetzte Basen, wie Triethylamin, Pyridin, N-Methylmorpholin oder N,N-Diethylanilin, als Lösungs- oder Verdünnungsmittel dienen.

Die Umsetzung erfolgt vorteilhaft in einem Temperaturbereich von etwa -30°C bis etwa +100°C, bevorzugt von etwa 0°C bis etwa +90°C.

In einer bevorzugten Ausführungsform der Variante e) wird eine Verbindung II bei -20° bis 20°, vorzugsweise 20°, in Gegenwart einer Base, vorzugsweise Pyridin, in einem halogenhaltigen Kohlenwasserstoff, vorzugsweise in Methylenchlorid, mit einem der in Variante e) genannten Acylierungsmittel und danach mit einem der in Variante d) genannten nukleophilen Reagenzien umgesetzt.

### Variante f):

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Mesitylen, Tetralin, Chlorbenzol, Dichlorbenzol, Brombenzol, Petrolether, Hexan, Cyclohexan, Dichlormethan, Trichlormethan, Tetrachlormethan, Dichlorethan, Trichlorethen oder Tetrachlorethen; Ether, wie Diethylether, Dipropylether, Diisopropylether, Dibutylether, tert.-Butylmethylether, Ethylenglykolmonomethylether, Ethylenglykolmonoethylether, Ethylenglykoldimethylether, Dimethoxydiethylether, Tetrahydrofuran oder Dioxan; und Sulfoxide, wie Dimethylsulfoxid.

Die Umsetzung erfolgt vorteilhaft in einem Temperaturbereich von etwa -30°C bis etwa +100°C, bevorzugt von etwa - 10°C bis etwa +20°C.

In einer bevorzugten Ausführungsform der Variante f) wird eine Verbindung II bei -20° bis 0°, vorzugsweise 0°, in Gegenwart einer Base, vorzugsweise Natriumhydrid, in einem Carbonsäureamid, vorzugsweise in Dimethylformamid, mit CS₂ und danach mit einem Alkylierungsmittel umgesetzt.

### Variante g):

Geeignete Basen zur Erleichterung der Umsetzung sind z. B. Alkylamine, Alkylendiamine, gegebenenfalls N-alkylierte, gegebenenfalls ungesättigte, Cycloalkylamine sowie basische Heterocyclen. Beispielhaft seien Triethylamin, Diisopropyl-ethyl-amin, Triethylendiamin, N-Cyclohexyl-N,N-dimethyl-amin, N,N-Diethylanilin, Pyridin, 4-(N,N-Dimethylamino)pyridin, Chinuclidin, N-Methylmorpholin wie 1,5-Diazabicyclo[5.4.0]undec-5-en (DBU) genannt.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Mesitylen, Tetralin, Chlorbenzol, Dichlorbenzol, Brombenzol, Petrolether, Hexan, Cyclohexan, Dichlormethan, Trichlormethan, Tetrachlormethan, Dichlorethan, Trichlorethen oder Tetrachlorethen; Ester, wie Essigsäureethylester; Ether, wie Diethylether, Dipropylether, Diisopropylether, Dibutylether, tert.-Butylmethylether, Ethylenglykolmonomethylether, Ethylenglykolmonoethylether, Ethylenglykoldimethylether, Dimethoxydiethylether, Tetrahydrofuran oder Dioxan; Ketone, wie Aceton, Methylethylketon oder Methylisobutylketon; Amide, wie N,N-Dimethylformamid, N,N-Diethylformamid, N,N-Dimethylacetamid, N-Methylpyrrolidon oder Hexamethylphosphorsäuretriamid; Nitrile, wie Acetonitril oder Propionitril; und Sulfoxide, wie Dimethylsulfoxid. Wird die Umsetzung in Gegenwart einer Base ausgeführt, können auch im Ueberschuss eingesetzte Basen, wie Triethylamin, Pyridin, N-Methylmorpholin oder N,N-Diethylanilin, als Lösungs- oder Verdünnungsmittel dienen.

Die Umsetzung erfolgt vorteilhaft in einen Temperaturbereich von etwa -30°C bis etwa +100°C, bevorzugt von etwa 0°C bis etwa +50°C.

In einer bevorzugten Ausführungsform der Variante g) wird eine Verbindung IV bei -20° bis 20°, vorzugsweise 20°, in Gegenwart einer Base, vorzugsweise Triethylamin, in einem Ether, vorzugsweise in Tetrahydrofuran, mit H₂S umgesetzt.

### Variante h):

Geeignete Abgangsgruppen sind z. B. Halogene oder C₁-C₈-Alkylsulfate, vorzugsweise Halogene, insbesondere Brom oder Iod, ganz besonders Iod.

Geeignete Basen zur Erleichterung der Umsetzung sind z. B. Alkalimetall- oder Erdalkalimetallhydroxide, -hydride, -amide, -alkanolate, -acetate, -carbonate, -dialkylamide oder -alkylsilylamide, Alkylamine, Alkylendiamine, gegebenenfalls N-alkylierte, gegebenenfalls ungesättigte, Cycloalkylamine, basische Heterocyclen, Ammoniumhydroxide sowie carbocyclische Amine. Beispielhaft seien Natriumhydroxid, -hydrid, -amid, -methanolat, -acetat, -carbonat, Kalium-tert.-butanolat, -hydroxid, -carbonat, -hydrid, Lithiumdiisopropylamid, Kalium-bis(trimethylsilyl)amid, Calciumhydrid, Triethylamin, Diisopropylethylamin, Triethylendiamin, Cyclohexylamin, N-Cyclohexyl-N,N-dimethylamin, N,N-Diethylanilin, Pyridin, 4-(N,N-Dimethylamino)pyridin, Chinuclidin, N-Methylmorpholin, Benzyltrimethylammoniumhydroxid sowie 1,5-Diazabicyclo[5.4.0]undec-5-en (DBU) genannt.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Mesitylen, Tetralin, Chlorbenzol, Dichlorbenzol, Brombenzol, Petrolether, Hexan, Cyclohexan, Dichlormethan, Trichlormethan, Tetrachlormethan, Dichlorethan, Trichlorethen oder Tetrachlorethen; Ester, wie Essigsäureethylester; Ether, wie Diethylether, Dipropylether, Diisopropylether, Dibutylether, tert.-Butylmethylether, Ethylenglykolmonomethylether, Ethylenglykolmonoethylether, Ethylenglykoldimethylether, Dimethoxydiethylether, Tetrahydrofuran oder Dioxan; Ketone, wie Aceton, Methylethylketon oder Methylisobutylketon; Alkohole, wie Methanol, Ethanol, Propanol, Isopropanol, Butanol, Ethylenglykol oder Glycerin; Amide, wie N,N-Dimethylformamid, N,N-Diethylformamid, N,N-Dimethylacetamid, N-Methylpyrrolidon oder Hexamethylphosphorsäuretriamid; Nitrile, wie Acetonitril oder Propionitril; und Sulfoxide, wie Dimethylsulfoxid. Wird die Umsetzung in Gegenwart einer Base ausgeführt, können auch im Ueberschuss eingesetzte Basen, wie Triethylamin, Pyridin, N-Methylmorphlin oder N,N-Diethylanilin, als Lösungs- oder Verdünnungsmittel dienen.

Die Umsetzung erfolgt vorteilhaft in einem Temperaturbereich von etwa -30°C bis etwa +100°C, bevorzugt von etwa 0°C bis etwa +90°C.

### Variante i):

Geeignete Säurekatalysatoren zur Erleichterung der Umsetzung sind z. B. Sulfonsäuren, wie Methan- oder p-Toluolsulfonsäure, Campher-10-Sulfonsäure, Pyridinio-p-toluolsulfonat, einschliesslich der sauren Ionenaustauscherharze mit Sulfogruppen, Lewis-Säuren, wie Bortrifluorid-Diethylether- oder -Dimethylether-Komplexe sowie Mineralsäuren, wie Schwefelsäure oder Phosphorsäure.

Geeignete wasserbindende Mittel zur Erleichterung der Wasser-Abspaltung sind z. B. Carbodiimide, wie N,N'-Dicyclohexylcarbodiimid, oder 1-Alkyl-2-halogen-pyridiniumsalze, wie 1-Methyl-2-chlor-pyridiniumiodid.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Mesitylen, Tetralin, Chlorbenzol, Dichlorbenzol, Brombenzol, Petrolether, Hexan, Cyclohexan, Dichlormethan, Trichlormethan, Tetrachlormethan, Dichlorethan, Trichlorethen oder Tetrachlorethen; Ester, wie Essigsäureethylester; Ether, wie Diethylether, Dipropylether, Diisopropylether, Dibutylether, tert.-Butylmethylether, Ethylenglykolmonomethylether, Ethylenglykolmonoethylether, Ethylenglykoldimethylether, Dimethoxydiethylether, Tetrahydrofuran oder Dioxan; Ketone, wie Aceton, Methylethylketon oder Methylisobutylketon; Alkohole, wie Methanol, Ethanol, Propanol, Isopropanol, Butanol, Ethylenglykol oder Glycerin; Amide, wie N,N-Dimethylformamid, N,N-Diethylformamid, N,N-Dimethylacetamid, N-Methylpyrrolidon oder Hexamethylphosphorsäuretriamid; Nitrile, wie Acetonitril oder Propionitril; und Sulfoxide, wie Dimethylsulfoxid. Wird die Umsetzung in Gegenwart eines Säurekatalysators ausgeführt, können auch im Ueberschuss eingesetzte Säuren, z. B. starke organische Carbonsäuren, wie gegebenenfalls, z. B. durch Halogen, substituierte C₁-C₄-Alkancarbonsäuren, z. B. Ameisensäure, Essigsäure oder Propionsäure, als Lösungs- oder Verdünnungsmittel dienen.

Die Umsetzung erfolgt vorteilhaft in einen Temperaturbereich von etwa -30°C bis etwa +100°C, bevorzugt von etwa 0°C bis etwa +90°C.

Salze von Verbindungen I können in an sich bekannter Weise hergestellt werden. So erhält man beispielsweise Säureadditionssalze von Verbindungen I durch Behandeln mit einer geeigneten Säure oder einem geeigneten Ionenaustauscherreagens und Salze mit Basen durch Behandeln mit einer geeigneten Base oder einem geeigneten Ionenaustauscherreagens.

Salze von Verbindungen I können in üblicher Weise in die freien Verbindungen I überführt werden, Säureadditionssalze z. B. durch Behandeln mit einem geeigneten basischen Mittel oder einem geeigneten Ionenaustauscherreagens und Salze mit Basen z. B. durch Behandeln mit einer geeigneten Säure oder einem geeigneten Ionenaustauscherreagens.

Salze von Verbindungen I können in an sich bekannter Weise in andere Salze von Verbindungen I umgewandelt werden, Säureadditionssalze beispielsweise in andere Säureadditionssalze, z. B. durch Behandeln eines Salzes einer anorganischen Säure, wie eines Hydrochlorids, mit einem geeigneten Metallsalz, wie einem Natrium-, Barium- oder Silbersalz, einer Säure, z. B. mit Silberacetat, in einem geeigneten Lösungsmittel, in welchem ein sich bildendes anorganisches Salz, z. B. Silberchlorid, unlöslich ist und damit aus dem Reaktionsgemisch ausscheidet.

Je nach Verfahrensweise bzw. Reaktionsbedingungen können die Verbindungen I mit salzbildenden Eigenschaften in freier Form oder in Form von Salzen erhalten werden.

Die Verbindungen I, II, III und IV können in Form eines der möglichen Isomeren oder als Gemisch derselben, z. B. je nach Anzahl, absoluter und relativer Konfiguration der asymmetrischen Kohlenstoffatome als reine Isomere, wie Antipoden und/oder Diastereomere, oder als Isomerengemische, wie Enantiomerengemische, z. B. Racemate, Diastereomerengemische oder Racematgemische, vorliegen; die Erfindung betrifft sowohl die reinen Isomeren als auch alle möglichen Isomerengemische und ist vor- und nachstehend jeweils entsprechend zu verstehen, auch wenn stereochemische Einzelheiten nicht in jedem Fall speziell erwähnt werden.

Verfahrensgemäss - je nach Wahl der Ausgangsstoffe und Arbeitsweisen - oder anderweitig erhältliche Diastereomerengemische und Racematgemische von Verbindungen I, II, III und IV können auf Grund der physikalisch-chemischen Unterschiede der Bestandteile in bekannter Weise in die reinen Diastereomeren oder Racemate aufgetrennt werden, beispielsweise durch fraktionierte Kristallisation, Destillation und/oder Chromatographie.

Entsprechend erhältliche Enantiomerengemische, wie Racemate, lassen sich nach bekannten Methoden in die optischen Antipoden zerlegen, beispielsweise durch Umkristallisation aus einem optisch aktiven Lösungsmittel, durch Chromatographie an chiralen Adsorbentien, z. B. Hochdruckflüssigkeitschromatographie (HPLC) an Acetylcellulose, mit Hilfe von geeigneten Mikroorganismen, durch Spaltung mit spezifischen, immobilisierten Enzymen, über die Bildung von Einschlussverbindungen, z. B. unter Verwendung chiraler Kronenether, wobei nur ein Enantiomeres komplexiert wird.

Ausser durch Auftrennung entsprechender Isomerengemische können reine Diastereomere bzw. Enantiomere erfindungsgemäss auch durch allgemein bekannte Methoden der diastereoselektiven bzw. enantioselektiven Synthese erhalten werden, z. B. indem man das erfindungsgemässe Verfahren mit Edukten mit entsprechend geeigneter Stereochemie ausführt.

Vorteilhaft isoliert bzw. synthetisiert man jeweils das biologisch wirksamere Isomere, z. B. Enantiomere, oder Isomerengemisch, z. B. Enantiomerengemisch, sofern die einzelnen Komponenten unterschiedliche biologische Wirksamkeit besitzen.

Die Verbindungen I, II, III und IV können auch in Form ihrer Hydrate erhalten werden und/oder andere, beispielsweise gegebenenfalls zur Kristallisation von in fester Form vorliegenden Verbindungen verwendete, Lösungsmittel einschliessen.

Die Erfindung betrifft alle diejenigen Ausführungsformen des Verfahrens, nach denen man von einer auf irgendeiner Stufe des Verfahrens als Ausgangs- oder Zwischenprodukt erhältlichen Verbindung ausgeht und alle oder einige der fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe und Zwischenprodukte verwendet, welche zu den eingangs als besonders wertvoll geschilderten Verbindungen I führen.

Die Erfindung betrifft insbesondere die in den Beispielen H1 bis H17 beschriebenen Herstellungsverfahren.

Erfindungsgemäss für die Herstellung der Verbindungen I bzw. ihrer Salze verwendete Ausgangsstoffe und Zwischenprodukte, jeweils in freier Form oder in Salzform, die neu sind, ihre Verwendung und Verfahren zu ihrer Herstellung bilden ebenfalls einen Gegenstand der Erfindung.

Die erfindungsgemässen Verbindungen I sind bei günstiger Warmblüter-, Fisch- und Pflanzenverträglichkeit wertvolle Wirkstoffe auf dem Gebiet der Schädlingsbekämpfung. Insbesondere wirken die erfindungsgemässen Wirkstoffe gegen Insekten, wie sie an Nutz- und Zierpflanzen in der Landwirtschaft und im Gartenbau, insbesondere in Baumwoll-, Gemüse- und Obstpflanzungen, und im Forst vorkommen. Die erfindungsgemässen Wirkstoffe eignen sich besonders zur Bekämpfung von Insekten in Obst- und Gemüsekulturen, insbesondere von pflanzenschädigenden Insekten, wie Spodoptera littoralis, Heliothis virescens, Diabrotica balteata und Crocidolomia binotalis. Weitere Anwendungsgebiete der erfindungsgemässen Wirkstoffe sind der Vorrats- und Materialschutz sowie im Hygienesektor insbesondere der Schutz von Haus- und Nutztieren. Die erfindungsgemässen Wirkstoffe sind gegen alle oder einzelne Entwicklungsstadien von normal sensiblen, aber auch von resistenten Arten von Schädlingen wirksam. Dabei kann sich ihre Wirkung z. B. in einer Abtötung der Schädlinge, welche unmittelbar oder erst nach einiger Zeit, beispielsweise bei einer Häutung, eintritt, oder in einer verminderten Eiablage und/oder Schlupfrate zeigen.

Zu den oben erwähnten Schädlingen gehören:
aus der Ordnung Lepidoptera zum Beispiel
Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula andalis, Hyphantria cunea, Keiferia lycopersicella, Leucoptera scitella, Lithocollethis spp., Lobesia botrana, Lymantria spp., Lyonetia spp., Malacosoma spp, Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;
aus der Ordnung Coleoptera zum Beispiel
Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp., Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;
aus der Ordnung Orthoptera zum Beispiel
Blatt spp., Blattella spp., Gryllotalpa spp., Leucophaea maderae, Locusta spp., Periplaneta spp. und Schistocerca spp.;
aus der Ordnung Isoptera zum Beispiel
Reticulitermes spp.;
aus der Ordnung Psocoptera zum Beispiel
Liposcelis spp.;
aus der Ordnung Anoplura zum Beispiel
Haematopinus spp., Linognathus spp., Pediculus spp., Pemphigus spp. und Phylloxera spp.;
aus der Ordnung Mallophaga zum Beispiel
Damalinea spp. und Trichodectes spp.;
aus der Ordnung Thysanoptera zum Beispiel
Frankliniella spp., Hercinothrips spp., Taeniothrips spp., Thrips palmi, Thrips tabaci und Scirtothrips aurantii;
aus der Ordnung Heteroptera zum Beispiel
Cimex spp., Distantiella theobroma, Dysdercus spp., Euchistus spp. Eurygaster spp. Leptocorisa spp., Nezara spp., Piesma spp., Rhodnius spp., Sahlbergella singularis, Scotinophara spp. und Triatoma spp.;
aus der Ordnung Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp`, Solenopsis spp. und Vespa spp.;
aus der Ordnung Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung Siphonaptera zum Beispiel
Ceratophyllus spp. und Xenopsylla cheopis und
aus der Ordnung Thysanura zum Beispiel
Lepisma saccharina.

Die gute pestizide Wirkung der erfindungsgemässen Wirkstoffe entspricht einer Abtötungsrate (Mortalität) von mindestens 50-60 % der erwähnten Schädlinge.

Die Wirkung der erfindungsgemässen Wirkstoffe und der sie enthaltenden Mittel lässt sich durch Zusatz von anderen Insektiziden wesentlich verbreitern und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die erfindungsgemässen Wirkstoffe werden in unveränderter Form oder vorzugsweise zusammen mit den in der Formulierungstechnik üblichen Hilfsmitteln eingesetzt und können daher beispielsweise zu emulgierbaren Konzentraten, direkt versprüh- oder verdünnbaren Lösungen, verdünnten Emulsionen, Spritzpulvern, löslichen Pulvern, Stäubemitteln und Granulaten, auch zu Verkapselungen in polymeren Stoffen, in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierungen, das heisst die den erfindungsgemässen Wirkstoff, beziehungsweise eine Kombination dieses Wirkstoffs mit anderen Insektiziden, und gegebenenfalls feste oder flüssige Hilfsstoffe enthaltenden Mittel, Zubereitungen oder Zusammensetzungen, werden in bekannter Weise hergestellt, zum Beispiel durch inniges Vermischen und/oder Vermahlen des Wirkstoffs mit den Hilfsstoffen, wie Streckmitteln, z. B. Lösungsmitteln oder festen Trägerstoffen, oder wie oberflächenaktiven Verbindungen (Tensiden).

Als Lösungsmittel können in Frage kommen: Aromatische Kohlenwasserstoffe, bevorzugt die Fraktionen C₈ bis C₁₂ von Alkylbenzolen, wie Xylolgemische oder alkylierte Naphthaline, aliphatische oder cycloaliphatische Kohlenwasserstoffe, wie Cyclohexan, Paraffine oder Tetrahydronaphthalin, Alkohole wie Ethanol, Propanol oder Butanol, Glykole sowie deren Ether und Ester, wie Propylenglykol, Dipropylenglykolether, Ethylenglykol, Ethylenglykolmonomethyl- oder -ethylether, Ketone, wie Cyclohexanon, Isophoron oder Diacetanolalkohol, stark polare Lösungsmittel, wie N-Methyl-2-pyrrolidon, Dimethylsulfoxid oder N,N-Dimethylformamid, Wasser sowie gegebenenfalls epoxidierte Pflanzenöle, wie gegebenenfalls epoxidiertes Raps-, Rizinus-, Kokosnuss- oder Sojaöl; gegebenenfalls auch Silikonöle.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, insbesondere Dolomit oder zerkleinerte Pflanzenrückstände, verwendet werden.

Als oberflächenaktive Verbindungen kommen je nach Art des zu formulierenden erfindungsgemässen Wirkstoffs oder der Kombination dieses Wirkstoffs mit anderen Insektiziden nichtionische, kationische und/oder anionische Tenside mit guten Emulgier-, Dispergier- und Netzeigenschaften in Betracht. Unter Tensiden sind auch Tensidgemische zu verstehen.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignet sind die wasserlöslichen 20 bis 250 Ethylenglykolethergruppen und 10 bis 100 Propylenglykolethergnippen enthaltenden Polyethylenoxid-Addukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykol-Einheiten. Als Beispiele nichtionischer Tenside seien Nonylphenolpolyethoxyethanole, Ricinusölpolyglykolether, Polypropylen-Polyethylenoxid-Addukte, Tributylphenoxypolyethoxyethanol, Polyethylenglykol und Octylphenoxypolyethoxyethanol erwähnt. Ferner kommen auch Fettsäureester von Polyoxyethylensorbitan, wie das Polyoxyethylensorbitan-trioleat, in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor; Beispiele sind das Stearyltrimethylammoniumchlorid oder das Benzyl-di-(2-chlorethyl)-ethylammoniumbromid.

Geeignete anionische Tenside können sowohl sogenannte wasserlösliche Seifen als auch wasserlösliche synthetische oberflächenaktive Verbindungen sein. Als Seifen eignen sich die Alkali-, Erdalkali- oder gegebenenfalls substituierten Ammoniumsalze von höheren Fettsäuren (C₁₀-C₂₂), wie die Natrium- oder Kalium-Salze der Oel- oder Stearinsäure oder von natürlichen Fettsäuregemischen, die beispielsweise aus Kokosnuss- oder Tallöl gewonnen werden können; ferner sind auch die Fettsäuremethyl-taurin-salze zu erwähnen. Häufiger werden jedoch sogenannte synthetische Tenside verwendet, insbesondere Fettsulfonate, Fettsulfate, sulfonierte Benzimidazolderivate oder Alkylarylsulfonate. Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst; beispielhaft genannt seien das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triethanolammoniumsalze der Dodecylbenzolsulfonsäure, der Dibutylnaphthalinsulfonsäure oder eines Naphthalinsulfonsäure-Formaldehyd-Kondensationsproduktes. Ferner kommen auch entsprechende Phosphate, wie Salze des Phosphorsäureesters eines p-Nonylphenol-(4-14)-Ethylenoxid-Adduktes oder Phospholipide, in Frage.

Die vorstehend aufgeführten Tenside sind nur als Beispiele anzusehen; in der einschlägigen Literatur werden viele weitere in der Formulierungstechnik gebräuchliche und erfindungsgemäss geeignete Tenside beschrieben.

Die pestiziden Zubereitungen enthalten in der Regel 0,1 bis 99%, insbesondere 0,1 bis 95%, an dem erfindungsgemässen Wirkstoff oder an der Kombination dieses Wirkstoffs mit anderen Insektiziden und 1 bis 99,9%, insbesondere 5 bis 99,9%, eines festen oder flüssigen Hilfsstoffes, wobei in der Regel 0 bis 25%, insbesondere 0,1 bis 20%, der Zubereitungen Tenside sein können (% bedeutet jeweils Gewichtsprozent). Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm Wirkstoff. Die Aufwandmengen pro Hektar betragen im allgemeinen 1 bis 1000 g Wirkstoff pro Hektar, vorzugsweise 25 bis 500 g/ha.

Insbesondere setzen sich bevorzugte Formulierungen folgendermassen zusammen (% = Gewichtsprozent):

| Emulgierbare Konzentrate: | |
|---|---|
| Wirkstoff | 1 bis 90 %, bevorzugt 5 bis 20 % |
| Tensid | 1 bis 30 %, vorzugsweise 10 bis 20 % |
| flüssiger Trägerstoff | 5 bis 94 %, vorzugsweise 70 bis 85 % |

| Stäube: | |
|---|---|
| Wirkstoff | 0,1 bis 10 %, vorzugsweise 0,1 bis 1 % |
| fester Trägerstoff | 99,9 bis 90 %, vorzugsweise 99,9 bis 99 % |

| Suspensions-Konzentrate: | |
|---|---|
| Wirkstoff | 5 bis 75 %, vorzugsweise 10 bis 50 % |
| Wasser | 94 bis 24 %, vorzugsweise 88 bis 30 % |
| Tensid | 1 bis 40 %, vorzugsweise 2 bis 30 % |

| Benetzbare Pulver: | |
|---|---|
| Wirkstoff | 0,5 bis 90 %, vorzugsweise 1 bis 80 % |
| Tensid | 0,5 bis 20 %, vorzugsweise 1 bis 15 % |
| fester Trägerstoff | 5 bis 95 %, vorzugsweise 15 bis 90 % |

| Granulate: | |
|---|---|
| Wirkstoff | 0,5 bis 30 %, vorzugsweise 3 bis 15 % |
| fester Trägerstoff | 99,5 bis 70 %, vorzugsweise 97 bis 85 % |

Die Zubereitungen können auch weitere Hilfsstoffe, wie Stabilisatoren, z. B. gegebenenfalls epoxidierte Pflanzenöle (z. B. epoxidiertes Kokosnussöl, Rapsöl oder Sojaöl), Entschäumer, z. B. Silikonöl, Konservierungsmittel, Viskositätsregulatoren, Bindemittel und/oder Haftmittel, sowie Dünger oder andere Wirkstoffe zur Erzielung spezieller Effekte enthalten.

Die folgenden Beispiele dienen der Erläuterung der Erfindung. Sie schränken die Erfindung nicht ein. Temperaturen sind in Grad Celsius angegeben.

### Herstellungsbeispiele

### Allgemeine Bemerkungen

Die Verbindungen fallen als Isomerengemische meistens in Form zäher Oele an. Sie können durch Säulenchromatographie gereinigt werden. NMR-Spektren und Elementaranalysen bestätigen die geforderten Strukturen.

### Beispiel H1: 4-Chlor-4'-trifluormethansulfonyloxybenzophenon-N-methylhydrazon

180 g 4-Chlor-4'-trifluormethansulfonyloxybenzophenon werden in 600 ml abs. Alkohol mit 29,6 g Eisessig und 31,8 g Methylhydrazin einen Tag lang am Rückfluss erhitzt. Dann wird die Lösung am Vakuum eingedampft, der Rückstand in ca. 150 ml Toluol aufgenommen und wieder eingedampft. Das verbleibende Oel wird in Hexan/Essigester 9:1 aufgenommen und die Lösung ca. 30 Minuten über Magnesiumsulfat und Natriumbikarbonat verrührt. Man filtriert von den Salzen ab und dampft das Filtrat am Vakuum ein. Es verbleibt die Titelverbindung als gelbes Oel.

### Beispiel H2: 4-Chlor-4'-trifluormethansulfonyloxybenzophenon-N-formyl-N-methylhydrazon (Verbindung 1.1 in Tabelle 1)

Unter Feuchtigkeitsausschuss werden 150 g Natriumformiat in ca. 125 ml Ether unter Rühren vorgelegt und tropfenweise mit 147 g (1,87 mol) Acetylchlorid bei 0°C Reaktionstemperatur versetzt. Man lässt mehrere Stunden bei Raumtemperatur nachrühren, filtriert vom Kochsalz ab und wäscht mit trockenem Ether nach. Man erhält so eine Lösung von etwa 1,87 Mol Formylacetat in Ether.
Ein Teil dieser Lösung, enthaltend 0,49 M Formylacetat, wird zu einer Lösung von 184 g des Hydrazons aus Beispiel H1 in 200 ml Ether getropft. Nach einigen Stunden ist die Reaktion beendet und man versetzt mit Eiswasser, sammelt die organische Schicht, wäscht nochmals mit Wasser und trocknet über Magnesiumsulfat, filtriert, dampft das Filtrat ein und reinigt durch Säulenchromatographie an Kieselgel mit Essigester/Hexan 1:9 als Laufmittel. Man erhält das Produkt als Isomerengemisch in Form einer ölig-wachsartigen Masse.

### Beispiel H3: 4-Chlor-4'-trifluormethansulfonyloxybenzophenon-N-acetyl-N-formylhydrazon (Verbindung 1.2 in Tabelle 1)

4,05 g 4-Chlor-4'-trifluormethansulfonyloxybenzophenon-N-formylhydrazon werden in 30 ml trockenem Tetrahydrofuran mit 0,7 ml Acetylchlorid und 1,4 ml Triethylamin verrührt, die Reaktionslösung wird nach 20 Stunden eingedampft und der Rückstand an einer Kieselgelsäule mit Essigester/Hexan 1:5 als Laufmittel chromatographiert. Man erhält die Titelverbindung als Isomerengemisch mit Smp. 137-142°C.

### Beispiel H4: 4-Chlor-4'-trifluormethansulfonyloxybenzophenon-N-chlorcarbonyl-N-methylhydrazon

Bei -10°C werden 57 ml 0,11 M Phosgenlösung in Toluol tropfenweise mit einer Lösung von 40,5 g des Hydrazons aus Beispiel H1 und 10,1 g Triethylamin in 100 ml Essigester versetzt. Nach ca. 1 Stunde wird die Reaktionslösung filtriert und mit etwas Essigester nachgewaschen, das Filtrat am Vakuum eingedampft und der Rückstand an einer Kieselgelsäule mit Essigester/Hexan 1:9 als Eluationsmittel gereinigt. Man erhält das Produkt als klebrige Kristallmasse.

### Beispiel H5: 4-Chlor-4'-trifluormethansulfonyloxybenzophenon-N-methyl-N-methylaminocarbonylhydrazon (Verbindung 1.3 in Tabelle 1)

0,01 M des Carbamoylchlorids aus Beispiel H4 als Rohlösung in Essigester werden mit 0,025 M Methylamin (33 % in abs. Alkohol) auf einmal versetzt. Nach 15 Minuten giesst man das Reaktionsgemisch auf Eiswasser, trennt die organische Schicht ab und dampft sie nach dem Trocknen über Magnesiumsulfat am Vakuum ein. Nach säulenchromatographischer Reinigung an Kieselgel mit Essigester/Hexan (1:2) als Laufmittel erhält man die Titelverbindung vom Smp. 112-116°C.

### Beispiel H6: 4-Chlor-4'-trifluormethansulfonyloxybenzophenon-N-methyl-N-thiomethylcarbonylhydrazon (Verbindung 1.4 in Tabelle 1)

0,02 M des Carbamoylchlorid aus Beispiel H4 in Essigester-Lösung werden mit 1,5 g Natriummethylmercaptid über Nacht verrührt, die Aufarbeitung ergibt die Titelverbindung als zähes, gelbliches Oel.

### Beispiel H7: 4-Chlor-4'-trifluormethansulfonyloxybenzophenon-N-methylthiomethylcarbonylhydrazon (Verbindung 1.126 in Tabelle 1)

2.55 g 4-Chlor-4'-trifluormethansulfonyloxybenzophenon-N-(1-chlor)-ethylidenhydrazon werden in 20 ml Tetrahydrofuran mit 0,065 Mol einer Lösung von Triethylammoniumhydrogensulfid in Tetrahydrofuran (zuvor hergestellt durch Einleiten von 34 g Schwefelwasserstoff in eine Lösung von 101 g Triethylamin in Tetrahydrofuran) versetzt. Man lässt einige Stunden nachrühren, dampft ein und chromatographiert den Rückstand am Kieselgel mit Essigester/Hexan 1:9 als Laufmittel. Man erhält ein Isomerengemisch der Titelverbindung als gelbliches, dichtes Oel.

### Beispiel H8: 4-Chlor-4'-trifluormethansulfonyloxybenzophenon-N,N-dimethylaminothiocarbonyl-N-methylhydrazon (Verbindung 1.127 in Tabelle 1)

Analog Beispiel H4 wird unter Verwendung von Thiophosgen anstelle von Phosgen eine gelbe Lösung von 4-Chlor-4'-trifiuormethansulfonyloxybenzophenon-N-methyl-N-chlorthiocarbamoylhydrazon in Essigester hergestellt. Von dieser Lösung wird ein Aliquot, enthaltend 0,023 Mol des Thiocarbamoylchlorids, mit 6,5 g 33 %iger Dimethylamin-Lösung in abs. Alkohol versetzt. Nach drei Stunden versetzt man mit Eiswasser, trennt die organische Schicht ab, trocknet sie und dampft ein. Der Rückstand wird an einer Kieselgelsäule mit Essigester/Hexan 1:9 als Laufmittel gereinigt. Man erhält die Titelverbindung als gelbes zähes Oel.

### Beispiel H9: 4-Chlor-4'-trifluormethansulfonyloxybenzophenon-N-methyl-N-thioformylhydrazon (Verbindung 1.128 in Tabelle 1)

3,6 g der Verbindung aus Beispiel H2 werden mit 1,9 g Phosphorpentasulfid in 30 ml Toluol 20 Stunden verrührt. Zur Aufarbeitung versetzt man mit Eiswasser und Natriumbikarbonat, extrahiert mit Essigester, trocknet die organische Phase, dampft am Vakuum ein und chromatographiert den Rückstand mit Essigester/Hexan 1:9. Man erhält die Titelverbindung als orangefarbenes Oel.

### Beispiel H10: 4-Chlor-4'-trifluormethansulfonyloxybenzophenon-N-methyl-N-methylthiothiocarbonylhydrazon (Verbindung 1.129 in Tabelle 1)

7,1 g der Verbindung aus Beispiel H1 werden in einem Gemisch aus 25 ml trockenem Dimethylformamid und 1,3 ml Schwefelkohlenstoff vorgelegt. Bei 0°C werden 0,6 g Natriumhydrid (80 % in Oel dispergiert) zugefügt. Nach ca. 30 Minuten erfolgt die Zugabe von 1,3 ml Methyljodid. Nach zwei Stunden versetzt man mit Eiswasser und extrahiert mit Ether. Die organischen Phasen werden vereint, getrocknet und eingedampft. Man erhält ein zähes Oel, das an einer Kieselgelsäule mit Essigester/Hexan 1:9 gereinigt wird. Die Titelverbindung wird als Isomerengemisch mit Smp. 94-99°C erhalten.

### Beispiel H11: 4-Chlor-4'-trifluormethansulfonyloxybenzophenon-N-aminothiocarbonylhydrazon (Verbindung 1.130 in Tabelle 1)

15 g 4-Chlor-4'-trifluormethansulfonyloxybenzophenonhydrazon werden mit 3,5 g Ammoniumthiocyanat in 100 ml Alkohol verrührt und 4,5 g Methansulfonsäure zugetropft. Man lässt mehrere Stunden nachrühren, bis das Edukt im Dünnschichtchromatogramm nicht mehr nachweisbar ist. Durch Zugabe von Wasser wird das Produkt ausgefällt. Man filtriert ab, trocknet und kristallisiert aus Essigester/Hexan um. Man erhält so die Titelverbindung vom Smp. 94-96°C.

### Beispiel H12: 4-Fluor-4'-trifluormethansulfonyloxybenzophenon-N-isobutylidencarbonyl-N-methylbenzophenon (Verbindung 1.12 in Tabelle 1)

Zu einer Lösung von 3,38 g 4-Fluor-4'-trifluormethansulfonyloxybenzophenon-N-methylhydrazon (hergestellt in Analogie zu dem in Beispiel H1 beschriebenen Verfahren) in 50 ml Tetrahydrofuran werden nacheinander 1,28 g N-Ethyl-diisopropylamin und 1,47 g 3,3 Dimethylacrylsäurechlorid zugegeben und 15 Stunden bei Raumtemperatur gerührt. Das Reaktionsgemisch wird am Vakuum eingedampft. Der Rückstand wird zwischen Dichlormethan und Wasser verteilt. Die organische Phase wird über Natriumsulfat getrocknet, filtriert und eingedampft. Das Rohprodukt wird an Silicagel mit Hexan/Ethylacetat 10:1 chromatographiert. Man erhält so die Titelverbindung als gelbes Oel.

### Beispiel H13: 4-Chlor-4'-trifluormethansulfonyloxybenzophenon-N-methyl-N-(N'-methoxy-N'-methyl)aminocarbonylhydrazon (Verbindung 1.5 in Tabelle 1)

Zu einer Lösung von 3,2 g 4-Chlor-4'-trifluormethansulfonyloxybenzophenon-N-methylhydrazon in 50 ml Toluol werden nacheinander 0,91 g Triethylamin und 1,1 g N-Methyl-N-methoxycarbamoylchlorid zugegeben. Nach 2 Stunden Rückfluss wird abgekühlt und am Vakuum eingedampft. Der Rückstand wird zwischen Ethylacetat und Wasser verteilt. Die organische Phase wird über Natriumsulfat getrocknet, filtriert und eingedampft. Das Rohprodukt wird an Kieselgel mit Ethylacetat/Hexan 1:5 chromatographiert. So erhält man die Titelverbindung als bräunliches Oel.

### Beispiel H14: 4-Chlor-4'-trifluormethansulfonyloxybenzophenon-N-chlormethylcarbonylhydrazon

Zu einer Lösung von 22 g 4-Chlor-4'-trifluormethansulfonyloxy benzophenonhydrazon und 5,5 g Pyridin in 100 ml Methylenchlorid werden innert 30 Min. 7,2 g Chloracetylchlorid zugetropft. Das Reaktiongemisch wird 16 Stunden bei Raumtemperatur gerührt, dann auf 100 ml Wasser gegossen und mit 200 ml Methylenchlorid extrahiert. Die organische Phase wird mit ges. NaCl-Lösung gewaschen, mit MgSO₄ getrocknet und eingedampft. Das erhaltene Rohprodukt ergibt nach chromatographischer Reinigung an Kieselgel mit Hexan/Essigsäureethylester 3:1 die Titelverbindung als Feststoff mit einem Smp. 123-125°C.

### Beispiel H15: 4-Chlor-4'-thfluormethansulfonyloxybenzophenon-N-(6-chlor-6,6-difluor-2-oxa-3-oxohexancarbonyl)hydrazon (Verbindung 1.78 in Tabelle 1)

Ein Gemisch von 3 g des Produktes aus Beispiel H14, 0,1 g Natriumjodid, 1,0 g 4-Chlor-4,4-difluorbuttersäure und 0,67 g Triethylamin in 10 ml DMF wird während 16 Stunden auf 90°C erhitzt. Dann wird das Reaktionsgemisch auf Wasser gegossen und mit 100 ml Essigsäureethylester extrahiert. Die organische Phase wird mit ges. NaCl-Lösung gewaschen, mit MgSO₄ getrocknet und eingedampft. Das erhaltene Rohprodukt ergibt nach chromatographischer Reinigung an Kieselgel mit Methylenchlorid/2 % Methanol die Titelverbindung als amorphen Feststoff.

### Beispiel H16: 4-Chlor-4'-trifluormethansulfonyloxybenzophenon-N-(acetoxymethylencarbonyl)hydrazon (Verbindung 1.77 in Tabelle 1)

Ein Gemisch von 4 g des Produktes aus Beispiel H14, 0,13 g Natriumjodid und 0,72 g Natriumacetat in 10 ml DMF wird während 5 Stunden auf 50°C erhitzt. Anschliessend wird das Reaktionsgemisch auf Wasser gegossen und mit 100 ml Essigsäureethylester extrahiert. Die organische Phase wird mit ges. NaCl-Lösung gewaschen, mit MgSO₄ getrocknet und eingedampft. Der erhaltene Rückstand ergibt nach chromatographischer Reinigung an Kieselgel mit Hexan/Essigsäureethylester 1:1 die Titelverbindung als amorphen Feststoff.

### Beispiel H17: 4-Chlor-4'-trifluormethansulfonyloxybenzophenon-N-(1-imidazolyl)methylencarbonylhydrazon (Verbindung 1.80 in Tabelle 1)

Ein Gemisch von 3 g des Produktes aus Beispiel H14, 0,1 g Natriumjodid, 0,45 g Imidazol und 0,66 g Triethylamin in 10 ml DMF wird 10 Stunden auf 50°C erhitzt. Anschliessend wird das Reaktionsgemisch auf Wasser gegossen und mit 100 ml Essigsäureethylester extrahiert. Die organische Phase wird mit ges. NaCl-Lösung gewaschen, mit MgSO₄ getrocknet und eingedampft. Das erhaltene Rohprodukt ergibt nach chromatographischer Reinigung an Kieselgel mit Methylenchlorid/Methanol (1%) die Titelverbindung als amorphen Feststoff.

### Beispiel H18:

In analoger Weise wie in den Beispielen H2, H3, H5 bis H13 und H15 bis H17 beschrieben können auch die anderen in Tabellen 1 bis 4 aufgeführten Verbindungen hergestellt werden. In der Spalte "physik. Daten" dieser Tabellen bedeuten die Zahlen den Schmelzpunkt.

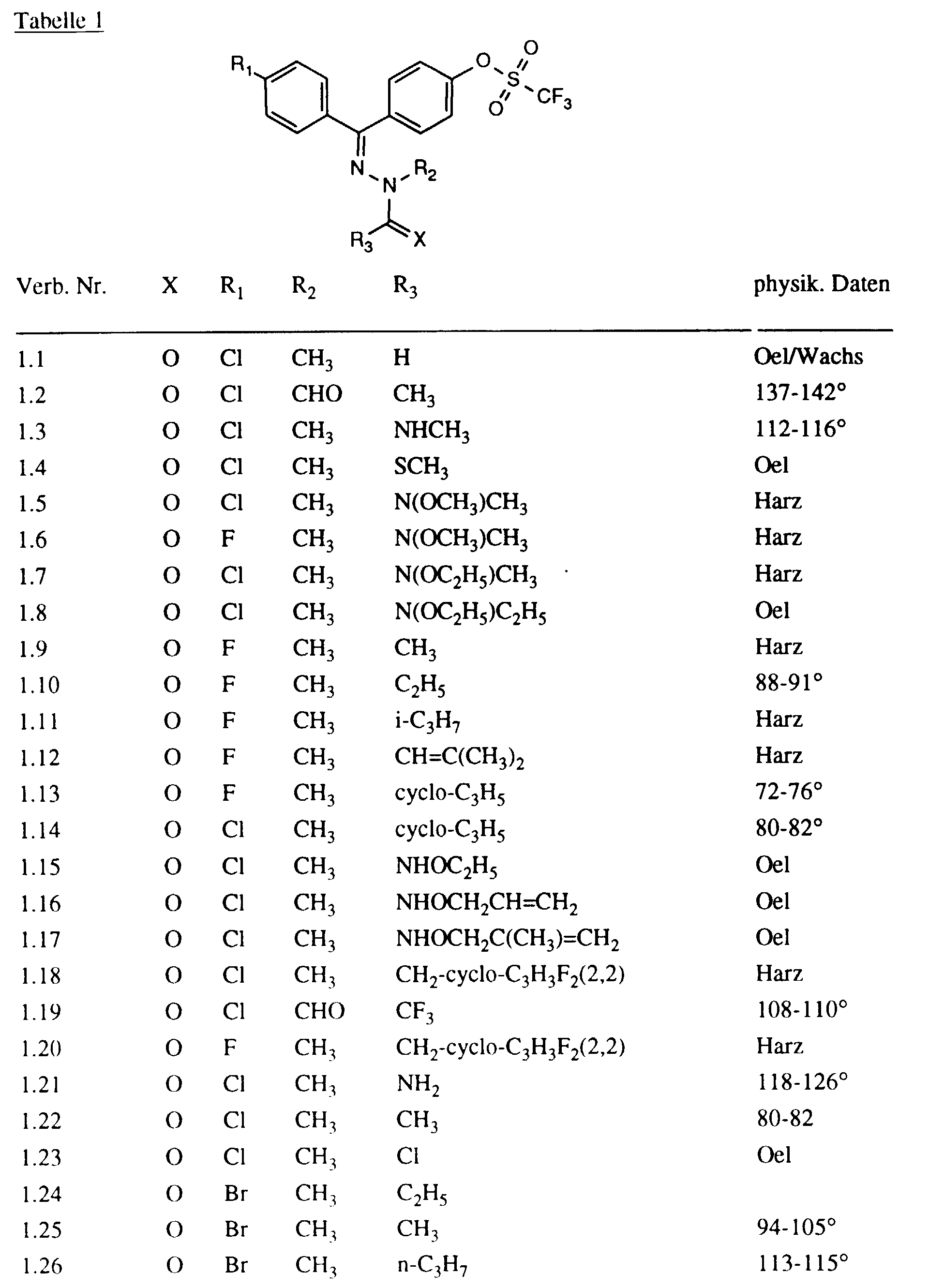

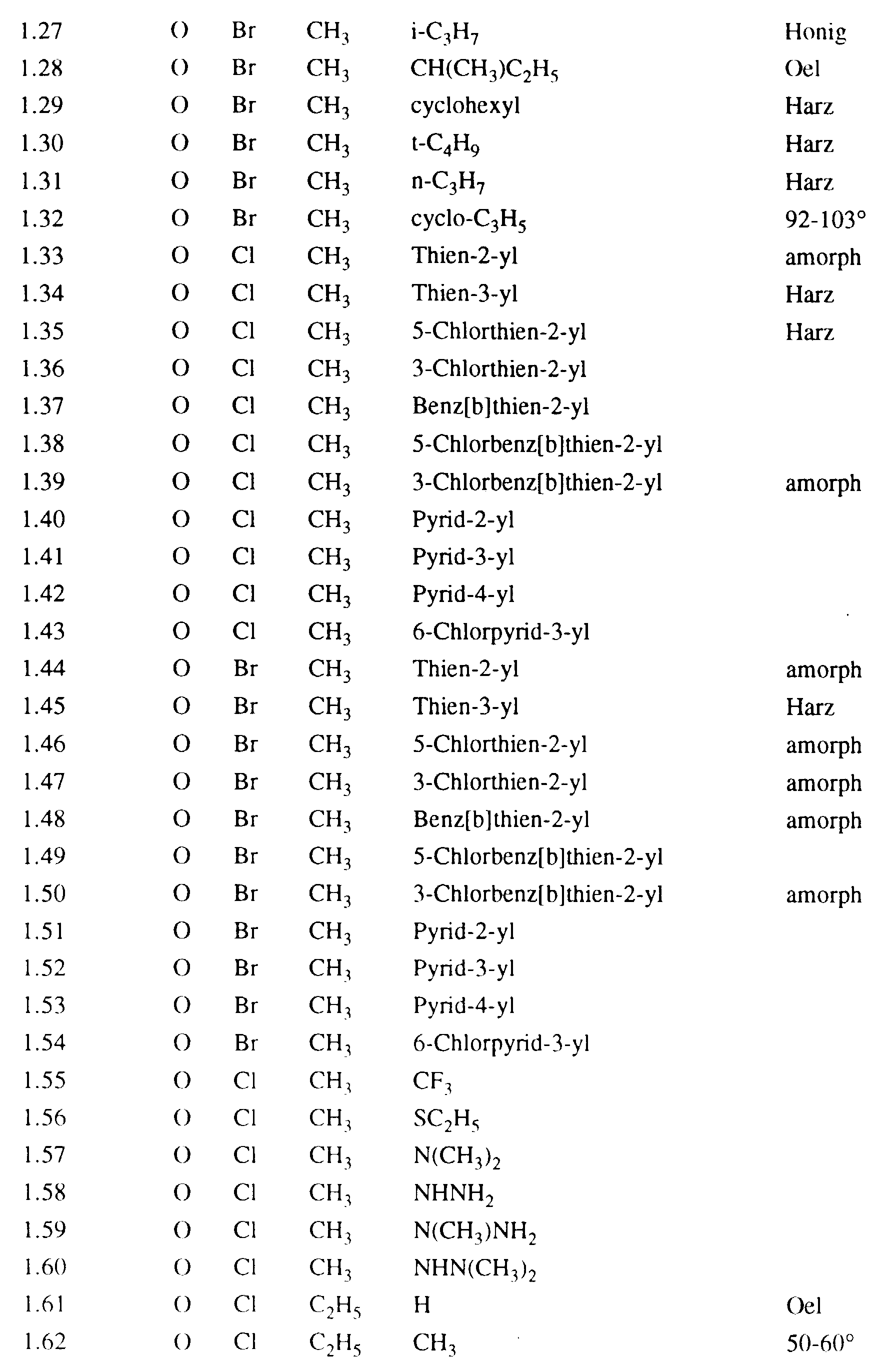

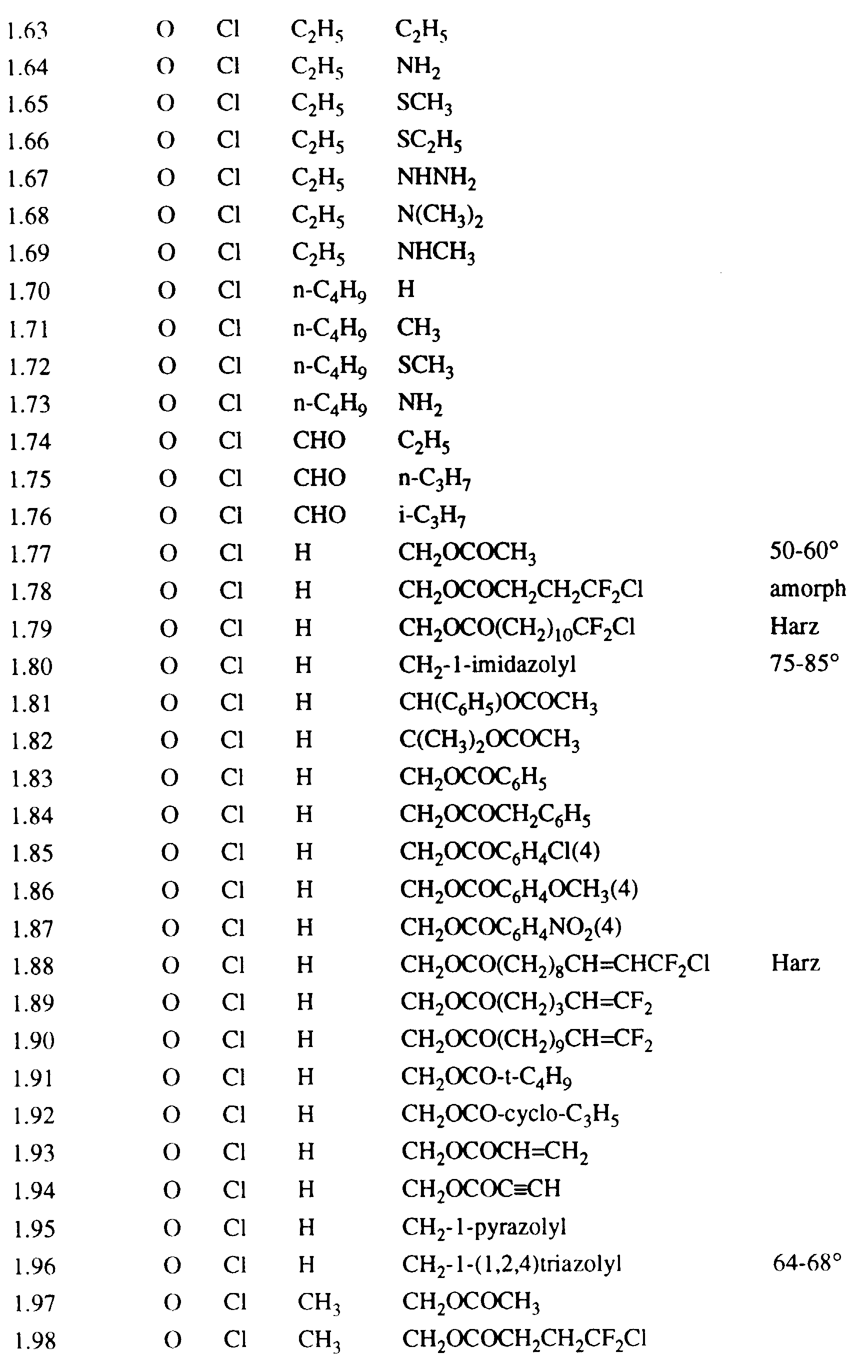

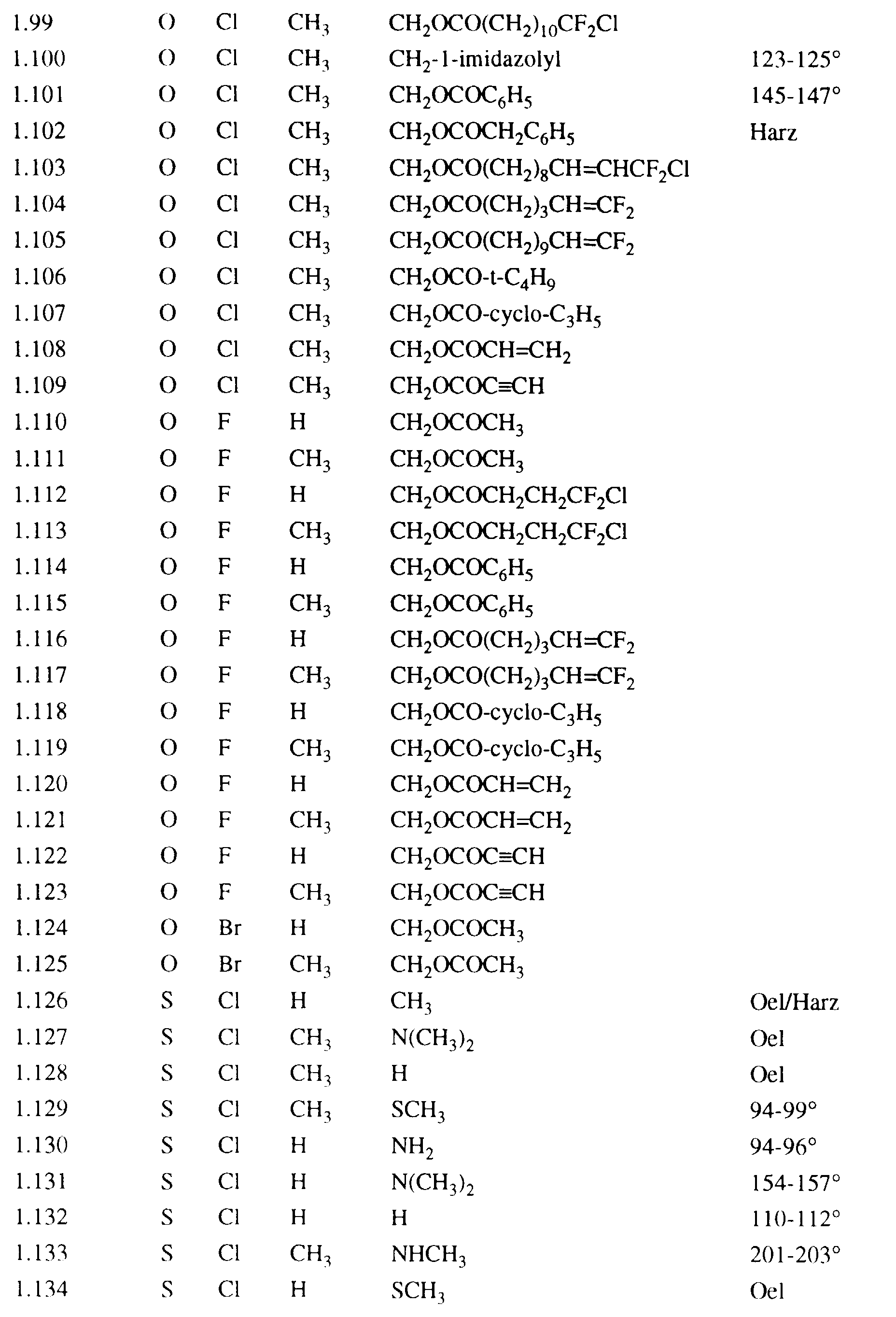

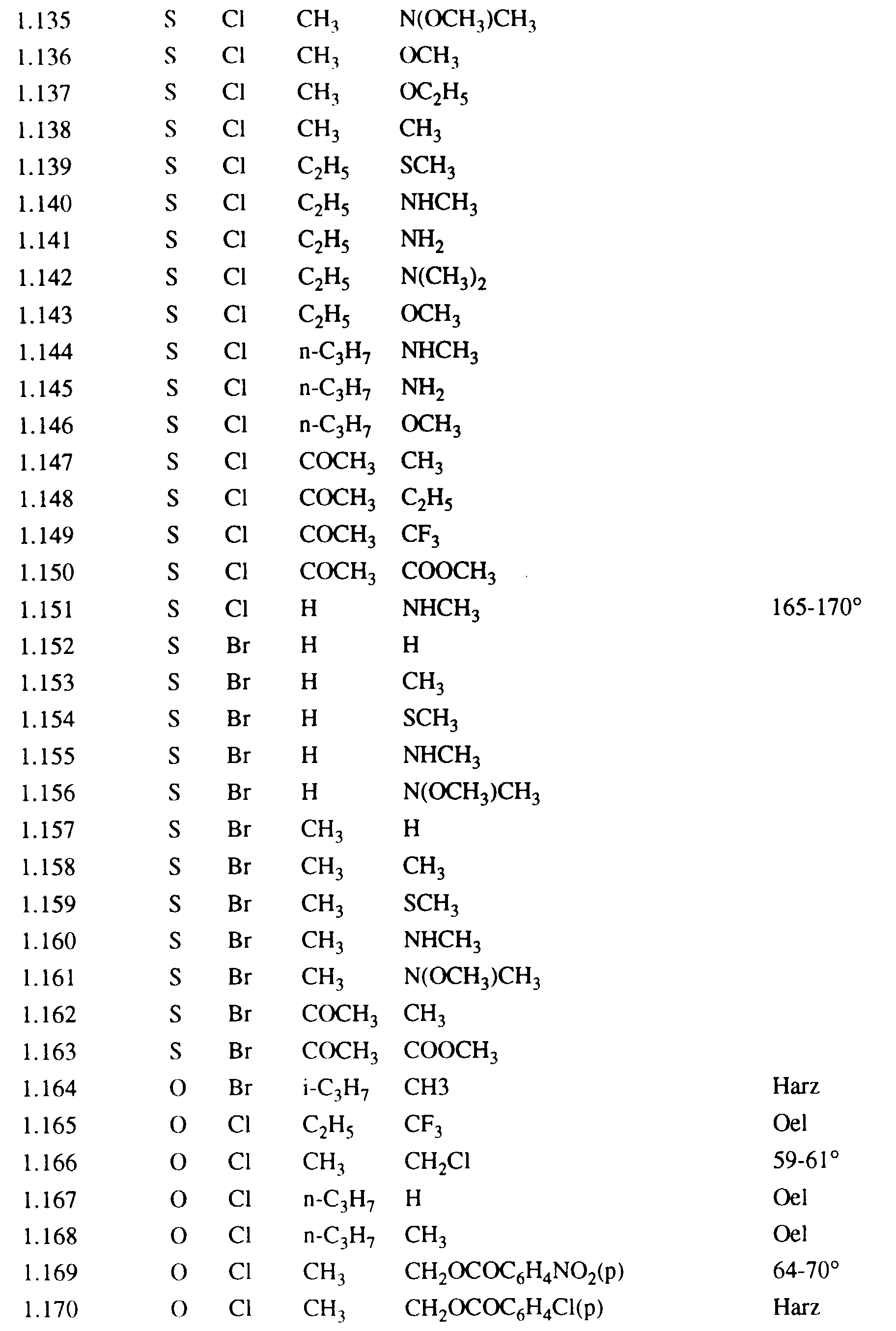

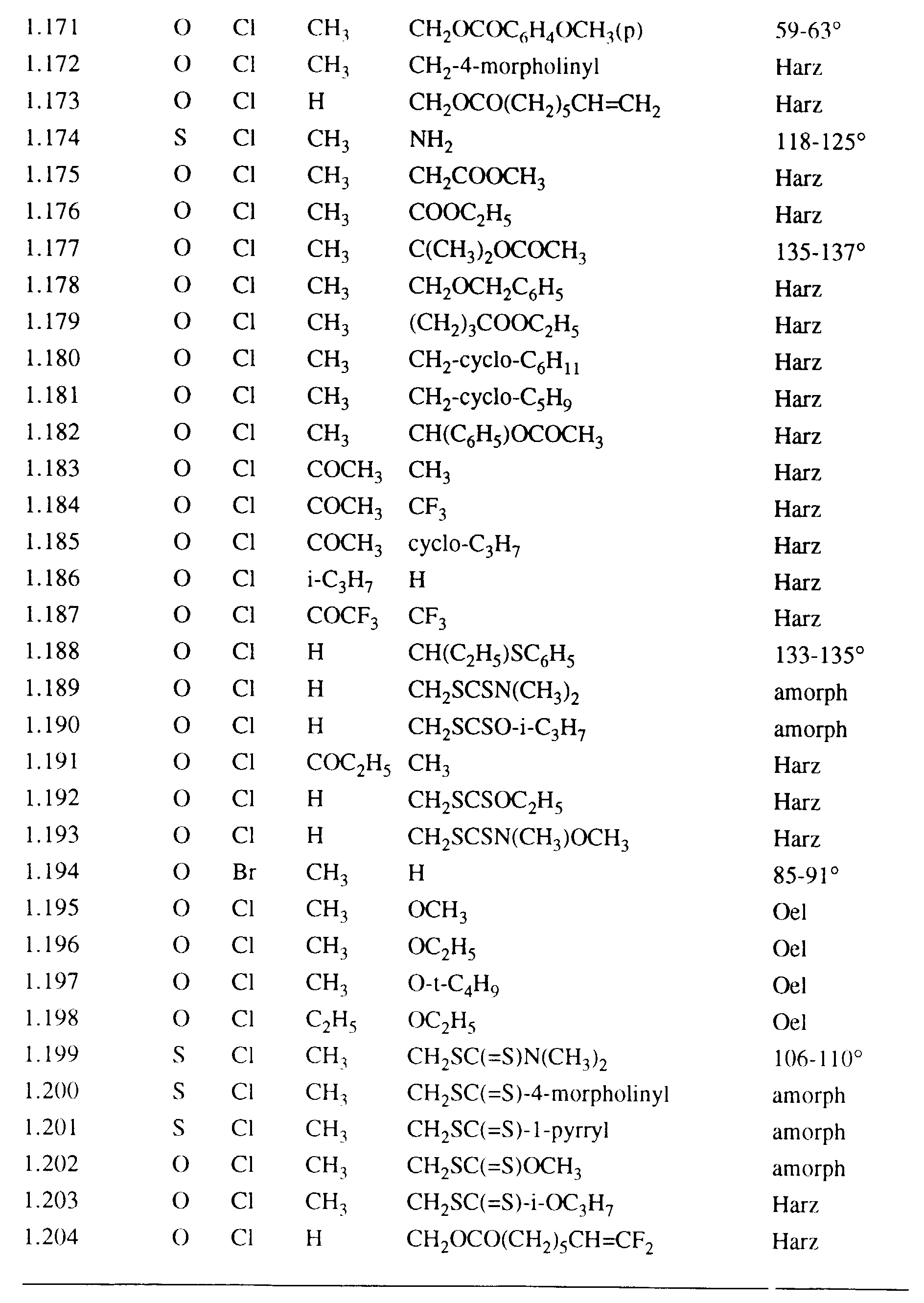

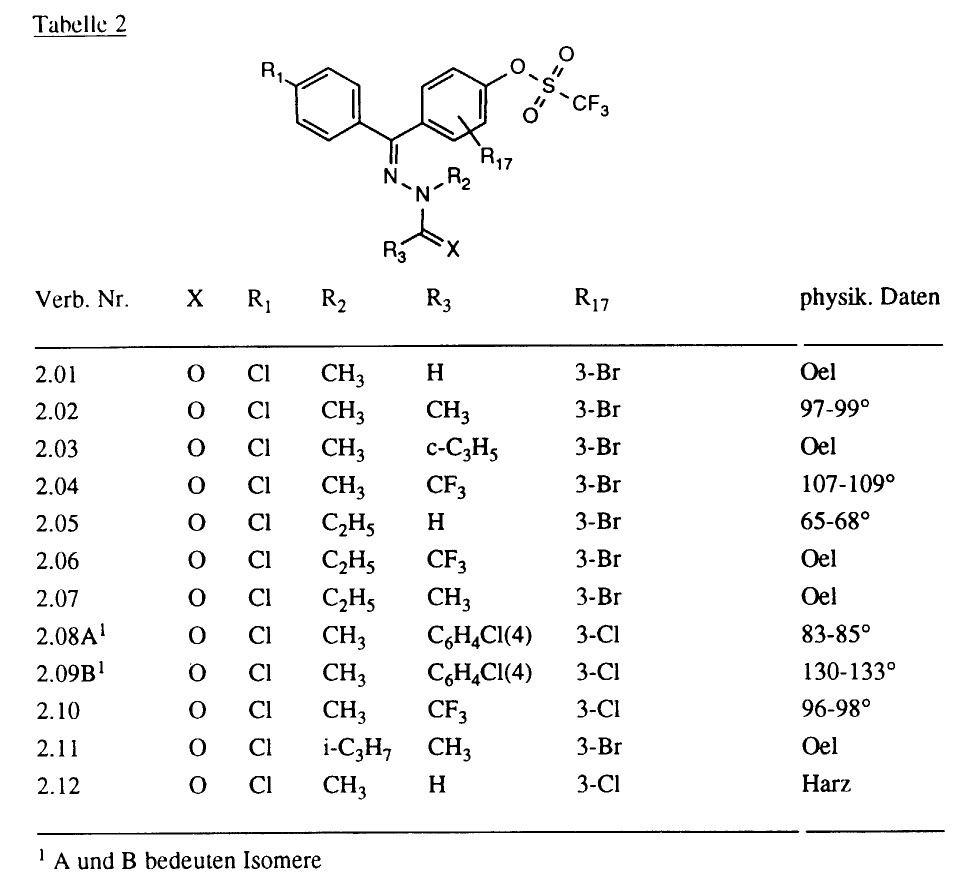

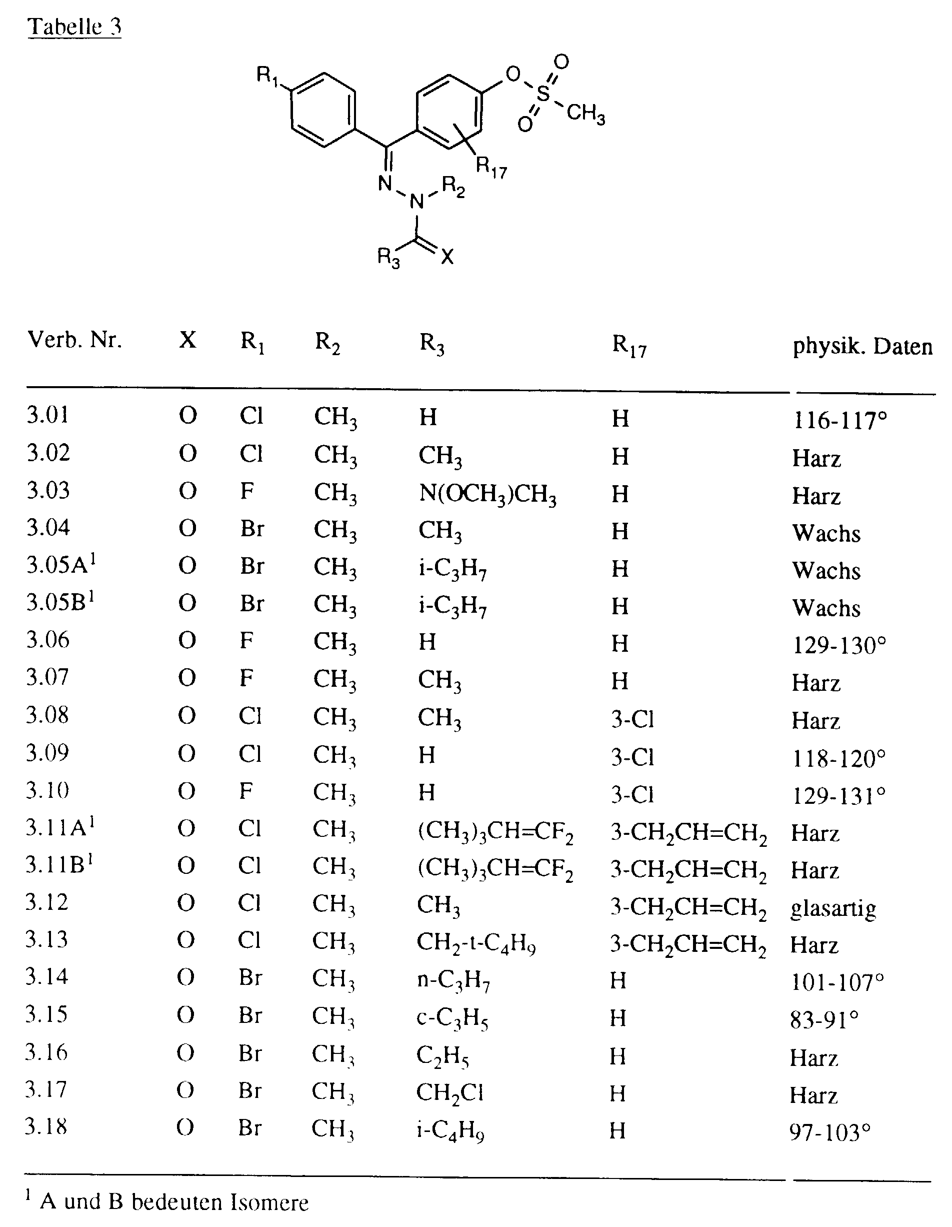

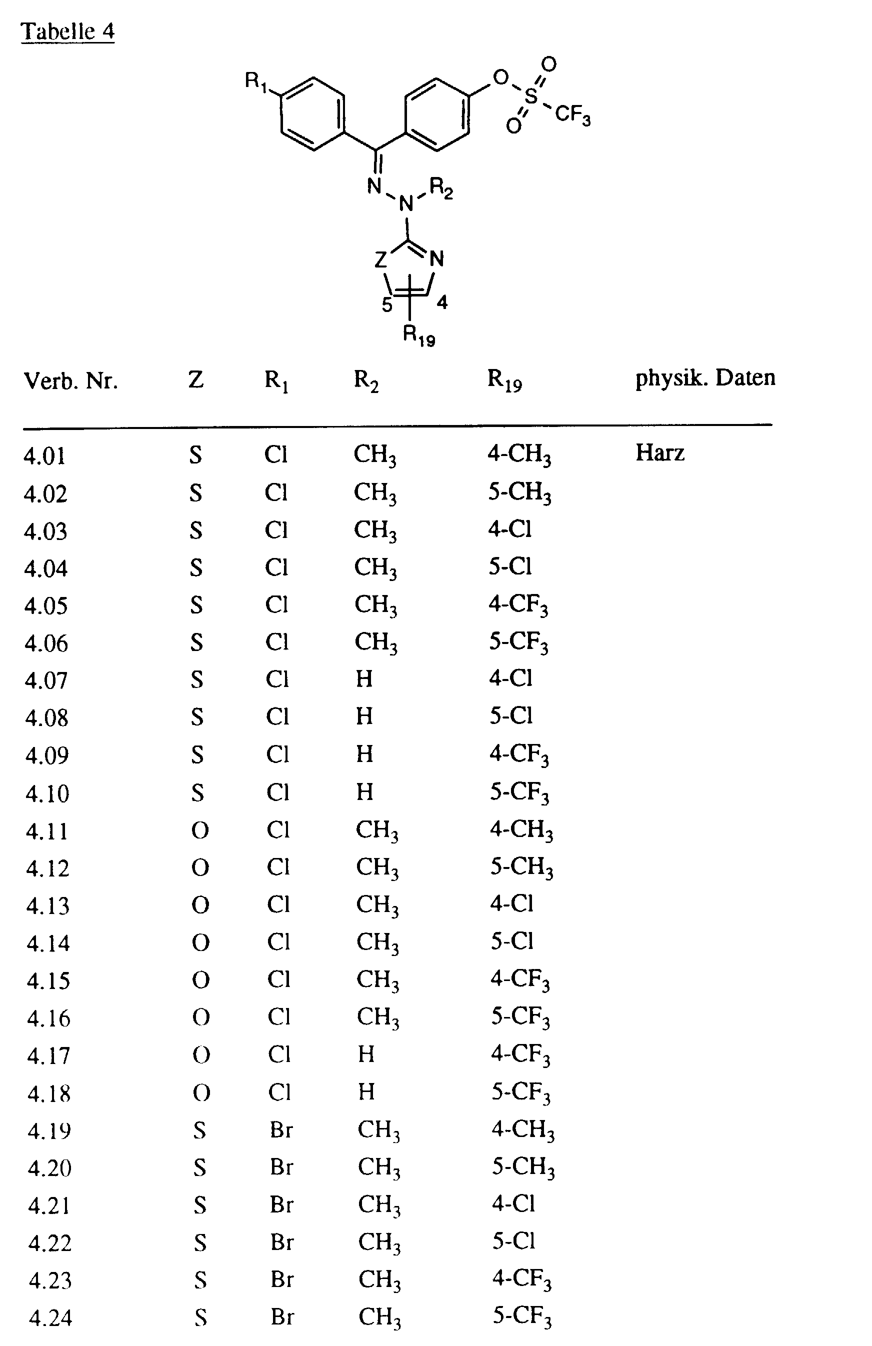

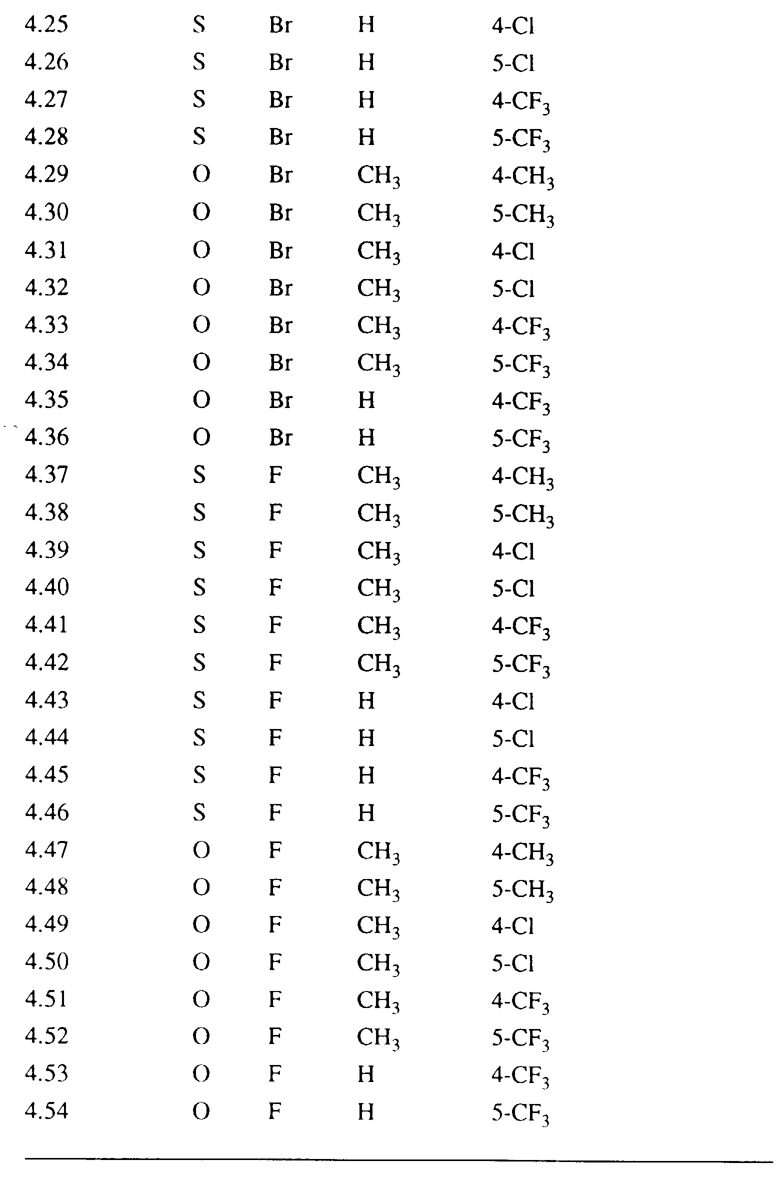

### Formulierungsbeispiele (% = Gewichtsprozent)

| Beispiel F1: Emulsions-Konzentrate | a) | b) | c) |
|---|---|---|---|
| Wirkstoff | 25% | 40% | 50% |
| Calciumdodecylbenzolsulfonat | 5% | 8% | 6% |
| Ricinusölpolyethylenglykolether (36 mol EO) | 5% | - | - |
| Tributylphenolpolyethylenglykolether (30 mol EO) | - | 12% | 4% |
| Cyclohexanon | - | 15% | 20% |
| Xylolgemisch | 65% | 25% | 20% |

Mischen von fein gemahlenem Wirkstoff und Zusatzstoffen ergibt ein Emulsions-Konzentrat, das durch Verdünnen mit Wasser Emulsionen gewünschter Konzentration liefert.

| Beispiel F2: Lösungen | a) | b) | c) | d) |
|---|---|---|---|---|
| Wirkstoff | 80% | 10% | 5% | 95% |
| Ethylenglykolmonomethylether | 20% | - | - | - |
| Polyethylenglykol (MG 400) | - | 70% | - | - |
| N-Methylpyrrolid-2-on | - | 20% | - | - |
| Epoxidiertes Kokosnussöl | - | - | 1% | 5% |
| Benzin (Siedegrenzen: 160-190°) | - | - | 94% | - |

Mischen von fein gemahlenem Wirkstoff und Zusatzstoffen ergibt eine Lösung, die zur Anwendung in Form Kleinster Tropfen geeignet ist.

| Beispiel F3: Granulate | a) | b) | c) | d) |
|---|---|---|---|---|
| Wirkstoff | 5% | 10% | 8% | 21% |
| Kaolin | 94% | - | 79% | 54% |
| Hochdisperse Kieselsäure | 1% | - | 13% | 7% |
| Attapulgit | - | 90% | - | 18% |

Der Wirkstoff wird in Dichlormethan gelöst, die Lösung auf das Trägerstoffgemisch aufgesprüht und das Lösungsmittel im Vakuum abgedampft.

| Beispiel F4: Stäubemittel | a) | b) |
|---|---|---|
| Wirkstoff | 2% | 5% |
| Hochdisperse Kieselsäure | 1% | 5% |
| Talkum | 97% | - |
| Kaolin | - | 90% |

Mischen von Wirkstoff und Trägerstoffen ergibt gebrauchsfertige Stäubemittel.

| Beispiel F5: Spritzpulver | a) | b) | c) |
|---|---|---|---|
| Wirkstoff | 25% | 50% | 75% |
| Natriumligninsulfonat | 5% | 5% | - |
| Natriumlaurylsulfat | 3% | - | 5% |
| Natriumdiisobutylnaphthalinsulfonat | - | 6% | 10% |
| Octylphenolpolyethylenglykolether (7-8 mol EO) | - | 2% | - |
| Hochdisperse Kieselsäure | 5% | 10% | 10% |
| Kaolin | 62% | 27% | - |

Wirkstoff und Zusatzstoffe werden gemischt und das Gemisch in einer geeigneten Mühle vermahlen. Man erhält Spritzpulver, die sich mit Wasser zu Suspensionen gewünschter Konzentration verdünnen lassen.

| Beispiel F6: Emulsions-Konzentrat | |
|---|---|
| Wirkstoff | 10% |
| Octylphenolpolyethylenglykolether (4-5 mol EO) | 3% |
| Calciumdodecylbenzolsulfonat | 3% |
| Ricinusölpolyethylenglykolether (36 mol EO) | 4% |
| Cyclohexanon | 30% |
| Xylolgemisch | 50% |

Mischen von fein gemahlenem Wirkstoff und Zusatzstoffen ergibt ein Emulsions-Konzentrat, das durch Verdünnen mit Wasser Emulsionen gewünschter Konzentration liefert.

| Beispiel F7: Stäubemittel | a) | b) |
|---|---|---|
| Wirkstoff | 5% | 8% |
| Talkum | 95% | - |
| Kaolin | - | 92% |

Man erhält anwendungsfertige Stäubemittel, indem man Wirkstoff und Trägerstoff mischt und das Gemisch in einer geeigneten Mühle vermahlt.

| Beispiel F8: Extruder-Granulat | |
|---|---|
| Wirkstoff | 10% |
| Natriumligninsulfonat | 2% |
| Carboxymethylcellulose | 1% |
| Kaolin | 87% |

Wirkstoff und Zusatzstoffe werden gemischt, das Gemisch vermahlen, mit Wasser angefeuchtet, extrudiert und granuliert und das Granulat im Luftstrom getrocknet.

| Beispiel F9: Umhüllungs-Granulat | |
|---|---|
| Wirkstoff | 3% |
| Polyethylenglykol (MG 200) | 3% |
| Kaolin | 94% |

Gleichmässiges Auftragen des fein gemahlenen Wirkstoffs auf das mit Polyethylenglykol angefeuchtete Kaolin in einem Mischer ergibt staubfreie Umhüllungs-Granulate.

| Beispiel F10: Suspensions-Konzentrat | |
|---|---|
| Wirkstoff | 40% |
| Ethylenglykol | 10% |
| Nonylphenolpolyethylenglykolether (15 mol EO) | 6% |
| Natriumligninsulfonat | 10% |
| Carboxymethylcellulose | 1% |
| wässrige Formaldehydlösung (37%) | 0,2% |
| wässrige Silikonölemulsion (75%) | 0,8% |
| Wasser | 32% |

Mischen von fein gemahlenem Wirkstoff und Zusatzstoffen ergibt ein Suspensions-Konzentrat, das durch Verdünnen mit Wasser Suspensionen gewünschter Konzentration liefert.

### Biologische Beispiele:

### Beispiel B1: Wirkung gegen Diabrotica balteata

Maiskeimlinge werden mit einer wässrigen Emulsionsspritzbrühe, enthaltend 400 ppm Wirkstoff, besprüht, nach Antrocknen des Spritzbelags mit 10 Larven des zweiten Stadiums von Diabrotica balteata besiedelt und dann in einen Plastikbehälter gegeben. Aus dem Vergleich der Anzahl toter Larven zwischen den behandelten und unbehandelten Pflanzen wird 6 Tage später die prozentuale Reduktion der Population (% Wirkung) bestimmt.
Verbindungen der Tabellen 1 bis 4 zeigen gute Wirkung in diesem Test. Insbesondere zeigen die Verbindungen Nr. 1.3, 1.5, 1.7 bis 1.11, 1.13, 1.15, 1.17, 1.19, 1.21 bis 1.23, 1.77 bis 1.79, 1.134, 1.151, 1.174 bis 1.177, 1.179, 1.183 bis 1.189, 1.191, 1.193 bis 1.196, 1.198, 2.01, 2.04 bis 2.07, 2.11, 2.12, 3.01 bis 3.08, 3.10, 3.15, 3.16 und 4.01 eine Wirkung von mehr als 80%.

### Beispiel B2: Wirkung gegen Heliothis virescens

Junge Sojapflanzen werden mit einer wässrigen Emulsionsspritzbrühe, enthaltend 400 ppm Wirkstoff, besprüht, nach Antrocknen des Spritzbelages mit 10 Raupen des ersten Stadiums von Heliothis virescens besiedelt und dann in einen Plastikbehälter gegeben. Aus den Vergleichen der Anzahl toter Raupen und des Frassschadens zwischen den behandelten und unbehandelten Pflanzen werden 6 Tage später die prozentuale Reduktion der Population und des Frasssschadens (% Wirkung) bestimmt.
Verbindungen der Tabellen 1 bis 4 zeigen gute Wirkung in diesem Test. Insbesondere zeigen die Verbindungen Nr. 1.1 bis 1.3, 1.5 bis 1.22, 1.77 bis 1.80, 1.126 bis 1.129, 1.132 1.134, 1.151, 1.176 bis 1.178, 1.183, 1.195, 1.196, 1.198, 2.11, 3.01, 3.02, 3.04 bis 3.07, 3.15, 3.16 und 4.01 eine Wirkung von mehr als 80%.

### Beispiel B3: Wirkung gegen Heliothis virescens (ovizid)

Auf Filterpapier abgelegte Eier von Heliothis virescens werden kurzzeitig in eine acetonisch-wässrige Lösung, enthaltend 400 ppm Wirkstoff, eingetaucht und nach Antrocknen des Tauchbelags in Petrischalen inkubiert. Nach 6 Tagen wird der prozentuale Schlupf der Eier im Vergleich zu unbehandelten Kontrollansätzen ausgewertet (% Schlupfreduktion).
Verbindungen der Tabellen 1 bis 4 zeigen gute Wirkung in diesem Test. Insbesondere zeigen die Verbindungen Nr. 1.1 bis 1.3, 1.5 bis 1.11, 1.13 bis 1.22, 1.77 bis 1.80, 1.126 bis 1.129, 1.132 bis 1.134, 1.151, 1.176 bis 1.178, 1.183, 1.195 bis 1.198, 2.01, 2.04 bis 2.06, 2.09, 2.11, 2.12, 3.01, 3.02, 3.04, 3.06, 3.07, 3.15, 3.16 und 4.01 eine Wirkung von mehr als 80%.

### Beispiel B4: Wirkung gegen Plutella xylostella

Junge Kohlpflanzen werden mit einer wässrigen Emulsionsspritzbrühe, enthaltend 400 ppm Wirkstoft besprüht, nach Antrocknen des Spritzbelags mit 10 Raupen des dritten Stadiums von Plutella xylostella besiedelt und dann in einen Plastikbehälter gegeben. Aus den Vergleichen der Anzahl toter Raupen und des Frassschadens zwischen den behandelten und unbehandelten Pflanzen werden 3 Tage später die prozentuale Reduktion der Population und die prozentuale Reduktion des Frassschadens (% Wirkung) bestimmt.
Verbindungen der Tabellen 1 bis 4 zeigen gute Wirkung in diesem Test. Insbesondere zeigen die Verbindungen Nr. 1.1 bis 1.3, 1.5 bis 1.11, 1.13 bis 1.22, 1.77 bis 1.80, 1.126 bis 1.129, 1.132 bis 1.134, 1.175 bis 1.178, 1.183, 1.195, 1.196, 1.198, 2.11, 3.01, 3.02, 3.04 bis 3.06, 3.14 bis 3.16 und 4.01 eine Wirkung von mehr als 80%.

### Beispiel B5: Wirkung gegen Spodoptera littoralis

Junge Sojapflanzen werden mit einer wässrigen Emulsionsspritzbrühe, enthaltend 400 ppm Wirkstoff, besprüht, nach Antrocknen des Spritzbelags mit 10 Raupen des dritten Stadiums von Spodoptera littoralis besiedelt und dann in einen Plastikbehälter gegeben. Aus den Vergleichen der Anzahl toter Raupen und des Frassschadens zwischen den behandelten und unbehandelten Pflanzen werden 3 Tage später die prozentuale Reduktion der Population und die prozentuale Reduktion des Frassschadens (% Wirkung) bestimmt.
Verbindungen der Tabellen 1 bis 4 zeigen gute Wirkung in diesem Test. Insbesondere zeigen die Verbindungen Nr. 1.1 bis 1.22, 1.77 bis 1.80, 1.126 bis 1.129, 1.132, 1.134, 1.175 bis 1.178, 1.183, 1.195 bis 1.198, 2.11, 3.01, 3.02, 3.04 bis 3.07, 3.14 bis 3.16 und 4.01 eine Wirkung von mehr als 80%.

### Beispiel B6: Wirkung gegen Boophilus microplus

Vollgesogene adulte Boophilus microplus Weibchen werden auf eine PVC Platte geklebt und mit einem Wattebausch überdeckt. Zur Behandlung werden die Testtiere mit 10 ml einer wässrigen Testlösung, die 125 ppm des zu prüfenden Wirkstoffes enthält, übergossen. Anschliessend wird der Wattebausch entfernt und die Zecken werden für 4 Wochen zur Eiablage inkubiert. Die Wirkung gegen Boophilus microplus zeigt sich entweder beim Weibchen als Mortalität oder Sterilität, oder bei den Eiern als ovizide Wirkung.
Verbindungen gemäss Tabellen 1 bis 4 zeigen in diesem Test gute Wirkung gegen Boophilus microplus. Insbesondere die Verbindung 1.204 zeigt eine Wirkung über 80 %.

### Beispiel B7: Wirkung gegen Blattella germanica

In eine Petri-Schale wird so viel einer 0,1 %igen acetonischen Lösung des Wirkstoffes gegeben, dass die Menge einer Aufwandmenge von 2 mg/m2 entspricht. Wenn das Lösungsmittel verdunstet ist, werden 20 Blattella germanica Nymphen (letztes Nymphenstadium) in die so vorbereitete Schale gegeben und 2 Stunden lang der Wirkung der Testsubstanz ausgesetzt. Dann werden die Nymphen mit CO2 narkotisiert, in eine frische Peth-Schale gebracht und im Dunkeln bei 25°C und 50 bis 70 % Luftfeuchtigkeit gehalten. Nach 48 Stunden wird die insektizide Wirkung durch Ermittlung der Abtötungsrate bestimmt.
Verbindungen der Tabellen 1 bis 4 zeigen gute Wirkung gegen Blattella germanica in diesem Test. Insbesondere die Verbindungen 1.61, 1.186 und 3.03 zeigen eine Wirkung über 80 %.

### Beispiel B8: Wirkung gegen Musca domestica

Ein Zuckerwürfel wird mit so viel Testsubstanz-Lösung behandelt, dass nach Trocknen über Nacht die Konzentration der Testsubstanz im Zucker 250 ppm beträgt. Der so behandelte Würfel wird mit einem nassen Wattebausch und 10 Adulten eines OP-resistenten Stammes von Musca domestica auf eine Aluminiumschale gelegt. Diese wird mit einem Becherglas abgedeckt und bei 25° inkubiert. Nach 24 Stunden wird die Mortalitätsrate bestimmt.
Verbindungen der Tabellen 1 bis 4 zeigen gute Wirkung in diesem Test. Insbesondere die Verbindungen 1.61, 1.186 und 3.03 zeigen eine Wirkung über 80 %.

## Patentansprüche

1. Eine Verbindung der Formel 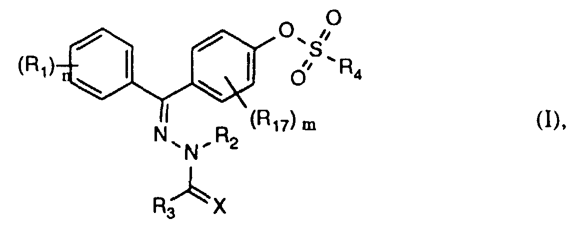 worin
m 0, 1, 2, 3 oder 4, wobei, wenn m grösser als 1 ist, die Reste R₁₇ gleich oder verschieden sind;
n 0, 1, 2, 3, 4 oder 5, wobei, wenn n grösser als 1 ist, die Reste R₁ gleich oder verschieden sind;
R₁ und R₁₇ unabhängig voneinander Halogen, C₁-C₈-Alkyl, Halogen-C₁-C₈-Alkyl, Phenoxy, Phenylthio, Halogen-C₁-C₈-Alkylthio, Phenyl, C₁-C₈-Alkylthio, C₁-C₈-Alkylcarbonyl, OSO₂R₄, SO₂NR₁₃R₁₄ oder P(=Y)R₂₂R₂₃;
entweder X S, R₂ R₂₁ oder H und R₃ R₃₁, -NR₅R₆ oder -OR₂,
oder X O, R₂ R₂₁ und R₃ R₃₁,
oder X O, R₂ H und R₃ -R₈OR₉, -R₈OCOR₉, -R₈NR₁₀R₁₁ oder -R₈R₁₂,
oder X und R₃ gemeinsam R₁₈;
Y O oder S;
R₄ C₁-C₈-Alkyl, Halogen-C₁-C₈-Alkyl oder Halogen-C₁-C₈-Alkoxy;
R₂₁ C₁-C₈-Alkyl, C₃-C₆-Cycloalkyl, Halogen-C₁-C₈-alkyl, C₁-C₄-Alkoxy-C₁-C₄-alkyl, C₁-C₄-Alkylthio-C₁-C₄-alkyl, R₁₃R₁₄N-C₁-C₄-alkyl, C₂-C₈-Alkenyl, C₂-C₈-Alkinyl, C₁-C₄-Acyl, CH=C(CN)₂ oder unsubstituiertes oder ein- bis fünffach substituiertes Phenyl, wobei die Substituenten ausgewählt sind aus der Gruppe, bestehend aus Halogen, C₁-C₄-Alkyl, C₂-C₅-Alkenyl, Halogen-C₁-C₄-alkyl, C₁-C₄-Alkoxy, SO₂NR₁₃R₁₄, Halogen-C₁-C₄-alkoxy, C₁-C₄-Alkylthio, C₁-C₄-Alkoxy-C₁-C₄-alkoxy, Halogen-C₁-C₄-alkylthio, NR₁₃R₁₄, -NO₂ und -CN;
R₃₁ H, C₁-C₈-Alkyl, C₃-C₆-Cycloalkyl, C₃-C₆-Cycloalkyl-C₁-C₃-alkyl, Halogen-C₁-C₈-alkyl, Halogen-C₃-C₆-Cycloalkyl-C₁-C₃-alkyl, C₂-C₈-Alkenyl, C₂-C₈-Alkinyl, Halogen-C₂-C₈-Alkenyl, -COOR₇, -SR₂, -R₈OR₉, -R₈OCOR₉, -R₈NR₁₀R₁₁, -R₈R₁₂, -R₈SR₉, -R₈SCSR₉, unsubstituiertes oder ein- bis fünffach substituiertes Phenyl, wobei die Substituenten ausgewählt sind aus der Gruppe, bestehend aus Halogen, C₁-C₄-Alkyl, C₂-C₅-Alkenyl, Halogen-C₁-C₄-alkyl, Halogen-C₁-C₄-alkoxy, Halogen-C₁-C₄-alkylthio, SO₂NR₁₃R₁₄, C₁-C₄-Alkoxy, -NO₂ und -CN; ein unsubstituierter oder substituierter, gesättigter oder ungesättigter, fünf- oder sechsgliedriger, heterocyclischer oder heterobicyclischer Ring, enthaltend ein bis drei Heteroatome, ausgewählt aus der Gruppe O, N und S, wobei die Substituenten ausgewählt sind aus der Gruppe, bestehend aus Halogen, C₁-C₄-Alkyl, Halogen-C₁-C₄-Alkyl und C₁-C₄-Alkoxy;
R₅ und R₆ unabhängig voneinander H, C₁-C₈-Alkyl, Halogen-C₁-C₈-Alkyl, C₂-C₅-Alkenyl, C₁-C₄-Alkoxy, C₂-C₅-Alkenoxy oder -NR₁₃R₁₄;
R₇ H, C₁-C₈-Alkyl, Halogen-C₁-C₈-Alkyl oder C₂-C₈-Alkenyl;
R₈ (CR₁₅R₁₆)ₚ, wobei p 1, 2, 3 oder 4 ist;
R₉ C₁-C₈-Alkyl, Halogen-C₁-C₁₁-Alkyl, C₃-C₆-Cycloalkyl, C₂-C₈-Alkenyl, Halogen-C₂-C₁₁-Alkenyl, C₂-C₈-Alkinyl, NR₁₀R₁₁, OR₁₀, Benzyl oder unsubstituiertes oder ein- bis fünffach substituiertes Phenyl, wobei die Substituenten ausgewählt sind aus der Gruppe, bestehend aus Halogen, C₁-C₄-Alkyl, C₂-C₅-Alkenyl, Halogen-C₁-C₄-alkyl, C₁-C₄-Alkoxy, -NO₂ und -CN;
R₁₀ und R₁₁ unabhängig voneinander C₁-C₈-Alkyl, C₂-C₈-Alkenyl oder C₁-C₈-Alkoxy-C₁-C₈-Alkyl;
R₁₂ ein unsubstituierter oder substituierter, gesättigter oder ungesättigter, fünf- oder sechsgliedriger, heterocyclischer oder heterobicyclischer Ring, enthaltend ein bis drei Heteroatome, ausgewählt aus der Gruppe O, N und S, wobei die Substituenten ausgewählt sind aus der Gruppe, bestehend aus Halogen, C₁-C₄-Alkyl, Halogen-C₁-C₄-alkyl, C₁-C₄-Alkoxy;
R₁₃ und R₁₄ unabhängig voneinander H oder C₁-C₈-Alkyl;
R₁₅ und R₁₆ unabhängig voneinander H, C₁-C₈-Alkyl oder unsubstituiertes oder ein- bis fünffach substituiertes Phenyl, wobei die Substituenten ausgewählt sind aus der Gruppe, bestehend aus Halogen, C₁-C₄-Alkyl, C₂-C₅-Alkenyl, Halogen-C₁-C₄-alkyl, C₁-C₄-Alkoxy, -NO₂ und -CN;
R₁₈ NC(R₁₉)=C(R₁₉)S, NC(R₁₉)=C(R₁₉)O, NC(R₁₉)₂C(R₁₉)₂S, NC(R₁₉)₂C(R₁₉)₂O, NC(R₁₉)=C(R₁₉)C(R₁₉)=N oder NC(R₁₉)=NC(R₁₉)=N;
R₁₉ H, C₁-C₄-Alkyl, Halogen oder Halogen-C₁-C₄-alkyl;
R₂₂ C₁-C₄-Alkyl, C₁-C₄-Alkoxy oder NR₁₃R₁₄; und
R₂₃ C₁-C₄-Alkyl, C₁-C₄-Alkoxy, C₃-C₄-Alkylthio oder NR₁₃R₁₄
bedeuten,
mit der Massgabe, dass in den Verbindungen der Formel I R₂ von H verschieden ist, wenn entweder m 0, n 1, R₁ 4-Cl, R₃ -N(OCH₃)CH₃, R₄ CF₃ und X S ist, oder m 0, n 2, R₁ 3,4-Dichlor, R₃ -N(OCH₃)CH₃, R₄ CF₃ und X S ist, oder m 0, n 1, R₁ 4-Cl, R₃ -NHNH₂, R₄ CF₃ und X S ist, oder gegebenenfalls ein Tautomeres davon, jeweils in freier Form oder in Salzform.

2. Eine Verbindung gemäss Anspruch 1 der Formel I, worin
m 0 oder 1 ist,
oder gegebenenfalls ein Tautomeres davon.

3. Eine Verbindung gemäss Anspruch 1 der Formel I, worin
m 0;
n 1;
R₁ Fluor, Chlor oder Brom;
R₂ C₁-C₄-Alkyl, Formyl oder Acetyl;
R₃ H, C₁-C₄-Alkyl, C₃-C₆-Cycloalkyl, Halogen-C₁-C₂-alkyl, Halogen-Cyclopropylmethyl, C₂-C₄-Alkenyl, -COOR₇, -SR₂, -R₈-OCOR₉, -R₈-R₁₂ oder ein unsubstituierter oder substituierter, ungesättigter, fünf- oder sechsgliedriger, heterocyclischer oder heterobicyclischer Ring, enthaltend N oder S, wobei die Substituenten ausgewählt sind aus der Gruppe Fluor und Chlor;
R₄ Trifluormethyl;
R₇ Methyl oder Ethyl;
R₈ CR₁₅R₁₆;
R₉ C₁-C₄-Alkyl, Halogen-C₈-C₁₁-Alkyl, Cyclopropyl, Vinyl, Halogen-C₈-C₁₁-Alkenyl, Ethinyl oder unsubstituiertes oder einfach substituiertes Phenyl, wobei die Substituenten ausgewählt sind aus der Gruppe, bestehend aus Chlor, Methyl, Methoxy, und -NO₂;
R₁₂ ein unsubstituierter, ungesättigter, fünfgliedriger, heterocyclischer Ring, enthaltend ein bis drei Heteroatome, ausgewählt aus der Gruppe N und S; und
R₁₅ und R₁₆ unabhängig voneinander H, Methyl oder Phenyl ist, oder gegebenenfalls ein Tautomeres davon.

4. Eine Verbindung gemäss Anspruch 1 der Formel I, worin
m 0;
n 1;
R₁ Fluor, Chlor oder Brom;
R₂ C₁-C₄-Alkyl, Formyl oder Acetyl;
R₃ H, C₁-C₄-Alkyl, C₃-C₆-Cycloalkyl, Halogen-C₁-C₂-alkyl, Halogen-Cyclopropylmethyl, C₂-C₄-Alkenyl, -COOR₇, -SR₂, -R₈-OCOR₉, -R₈-R₁₂ oder ein unsubstituierter oder substituierter, ungesättigter, fünf- oder sechsgliedriger, heterocyclischer oder heterobicyclischer Ring, enthaltend N oder S, wobei die Substituenten ausgewählt sind aus der Gruppe Fluor und Chlor;
R₄ Trifluormethyl;
R₇ Methyl oder Ethyl;
R₈ CH₂, C(Methyl)₂ oder CH(Phenyl);
R₉ C₁-C₄-Alkyl, Halogen-C₈-C₁₁-Alkyl, Cyclopropyl, Vinyl, Halogen-C₈-C₁₁-Alkenyl, Ethinyl oder unsubstituiertes oder einfach substituiertes Phenyl, wobei die Substituenten ausgewählt sind aus der Gruppe, bestehend aus Chlor, Methyl, Methoxy, und -NO₂; und
R₁₂ ein unsubstituierter, ungesättigter, fünfgliedriger, heterocyclischer Ring, enthaltend ein bis drei Heteroatome, ausgewählt aus der Gruppe N und S, oder gegebenenfalls ein Tautomeres davon.

5. 4-Chlor-4'-trifluormethansulfonyloxybenzophenon-N-formyl-N-methylhydrazon gemäss Anspruch 1.

6. Verfahren zur Herstellung einer Verbindung gemäss Anspruch 1 der Formel I oder gegebenenfalls eines Tautomeren davon, jeweils in freier Form oder in Salzform, unter Berücksichtigung der vorstehend erwähnten Massgabe, dadurch gekennzeichnet, dass man
a) eine Verbindung der Formel 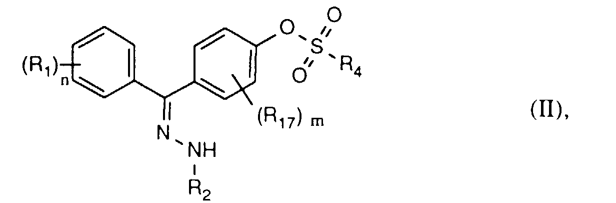 die bekannt ist oder in Analogie zu entsprechenden bekannten Verbindungen hergestellt werden kann und worin m, n, R₁, R₂, R₄ und R₁₇ die für die Formel I angegebenen Bedeutungen haben, oder gegebenenfalls ein Tautomeres davon, in freier Form oder in Salzform, vorzugsweise in Gegenwart einer Base, mit Phosgen oder Thiophosgen umsetzt und das gegebenenfalls isolierte Zwischenprodukt, eventuell in Gegenwart einer Base, mit einer Verbindung der Formel Y₁H, worin Y₁ -NR₅R₆, -OR₂ oder -SR₂ ist und R₂, R₅ und R₆ die für die Formel I angegebenen Bedeutungen haben, oder einem Salz davon, umsetzt oder
b) zur Herstellung einer Verbindung der Formel I, worin X O ist, eine Verbindung der Formel II, eventuell in Gegenwart einer Base, mit einer Verbindung der Formel R₃COY₂, worin R₃ die für die Formel I angegebenen Bedeutungen mit Ausnahme von -OR₂ und -SR₂ hat und Y₂ eine Abgangsgruppe ist, umsetzt oder
c) zur Herstellung einer Verbindung der Formel I, worin X S ist, eine Verbindung der Formel II mit einer Verbindung der Formel Y₃-N=C=S, worin Y₃ H, C₁-C₈-Alkyl, Halogen-C₁-C₈-Alkyl oder C₂-C₅-Alkenyl, bevorzugt H oder C₁-C₈-Alkyl, bedeutet, zu Verbindungen der Formel I, worin X S ist, umsetzt oder
d) zur Herstellung einer Verbindung der Formel I, worin X S und R₃ -SR, worin R C₁-C₈-Alkyl, C₃-C₆-Cycloalkyl, C₂-C₈-Alkenyl oder C₂-C₈-Alkinyl, bevorzugt C₁-C₈-Alkyl, C₃-C₆-Cycloalkyl, insbesondere C₁-C₈-Alkyl bedeutet, ist, eine Verbindung der Formel II, eventuell in Gegenwart einer Base, mit CS₂ umsetzt und das gegebenenfalls isolierte Zwischenprodukt, eventuell in Gegenwart einer Base, mit einer Verbindung der Formel RY₄, worin R die oben definierten Bedeutungen hat und Y₄ eine Abgangsgruppe ist, umsetzt oder
e) zur Herstellung einer Verbindung der Formel I, worin X O ist, eine Verbindung der Formel II, eventuell in Gegenwart einer Base, mit einer Verbindung der Formel ClR₈COY₅, worin R₈ die für die Formel I angegebenen Bedeutungen hat und Y₅ eine Abgangsgruppe ist, umsetzt und das entstandene Zwischenprodukt, eventuell in Gegenwart einer Base, eventuell in Gegenwart eines Alkalimetallhalogenids, wie in Gegenwart von Natriumiodid, mit einer Verbindung der Formel R₉COOH, HNR₁₀R₁₁ oder R₁₂, worin R₉, R₁₀, R₁₁ und R₁₂ die für die Formel I angegebenen Bedeutungen haben, umsetzt oder
f) zur Herstellung einer Verbindung der Formel I, worin X S ist, eine Verbindung der Formel 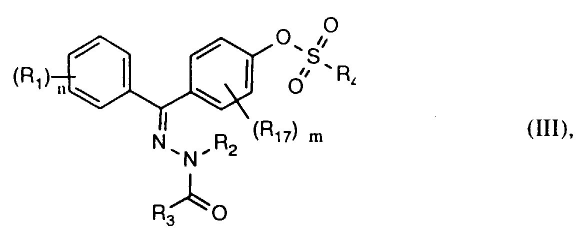 die bekannt ist oder in Analogie zu entsprechenden bekannten Verbindungen, z. B. nach der Variante b), hergestellt werden kann und worin m, n, R₁, R₂, R₃, R₄ und R₁₇ die für die Formel I angegebenen Bedeutungen haben, mit P₂S₅ umsetzt, oder
g) zur Herstellung einer Verbindung der Formel I, worin X S und R₂ H ist, eine Verbindung der Formel 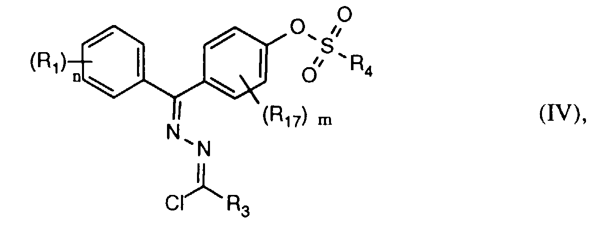 die bekannt ist oder in Analogie zu entsprechenden bekannten Verbindungen hergestellt werden kann und worin m, n, R₁, R₃, R₄ und R₁₇ die für die Formel I angegebenen Bedeutungen haben, eventuell in Gegenwart einer Base, mit H₂S umsetzt oder
h) eine Verbindung der Formel I, worin R₂ H ist, eventuell in Gegenwart einer Base, mit einer Verbindung der Formel R₂Y₆, worin R₂ die für die Formel I angegebenen Bedeutungen mit Ausnahme von H hat und Y₆ eine Abgangsgruppe ist, umsetzt oder
i) eine Verbindung der Formel 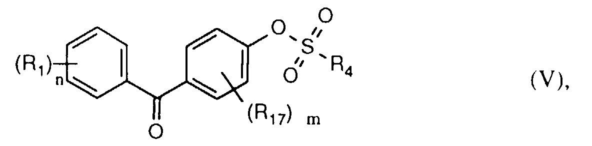 die bekannt ist oder in Analogie zu entsprechenden bekannten Verbindungen hergestellt werden kann und worin m, n, R₁, R₄ und R₁₇ die für die Formel I angegebenen Bedeutungen haben, eventuell in Gegenwart eines Säurekatalysators, eventuell in Gegenwart eines wasserbindenden Mittels, mit einer Verbindung der Formel 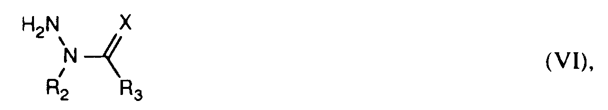 die bekannt ist oder in Analogie zu entsprechenden bekannten Verbindungen hergestellt werden kann und worin R₂, R₃ und X die für die Formel I angegebenen Bedeutungen haben, oder einem Salz und/oder gegebenenfalls einem Tautomeren davon umsetzt und jeweils, wenn erwünscht, eine verfahrensgemäss oder auf andere Weise erhältliche Verbindung der Formel I oder ein Tautomeres davon, jeweils in freier Form oder in Salzform, in eine andere Verbindung der Formel I oder ein Tautomeres davon überführt, ein verfahrensgemäss erhältliches Gemisch von Isomeren auftrennt und das gewünschte Isomere isoliert und/oder eine verfahrensgemäss erhältliche freie Verbindung der Formel I oder ein Tautomeres davon in ein Salz oder ein verfahrensgemäss erhältliches Salz einer Verbindung der Formel I oder eines Tautomeren davon in die freie Verbindung der Formel I oder ein Tautomeres davon oder in ein anderes Salz überführt.

7. Schädlingsbekämpfungsmittel, dadurch gekennzeichnet, dass es mindestens eine Verbindung gemäss Anspruch 1 der Formel I oder gegebenenfalls ein Tautomeres davon, jeweils in freier Form oder in agrochemisch verwendbarer Salzform, als Wirkstoff und gegebenenfalls mindestens einen Hilfsstoff enthält.

8. Verfahren zur Herstellung eines mindestens einen Hilfsstoff enthaltenden Mittels gemäss Anspruch 7, dadurch gekennzeichnet, dass man den Wirkstoff mit dem (den) Hilfsstoff(en) innig vermischt und/oder vermahlt.

9. Verwendung einer Verbindung gemäss Anspruch 1 der Formel I oder gegebenenfalls eines Tautomeren davon, jeweils in freier Form oder in agrochemisch verwendbarer Salzform, zur Herstellung eines Mittels gemäss Anspruch 7.

10. Verwendung eines Mittels gemäss Anspruch 7 zur Bekämpfung von Schädlingen.

11. Verwendung gemäss Anspruch 10 zum Schutz von pflanzlichem Vermehrungsgut.

12. Verfahren zur Bekämpfung von Schädlingen, dadurch gekennzeichnet, dass man ein Mittel gemäss Anspruch 7 auf die Schädlinge oder ihren Lebensraum appliziert.

13. Verfahren gemäss Anspruch 12 zum Schutz von pflanzlichem Vermehrungsgut, dadurch gekennzeichnet, dass man das Vermehrungsgut oder den Ort der Ausbringung des Vermehrungsguts behandelt.

14. Pflanzliches Vermehrungsgut, behandelt gemäss dem in Anspruch 13 beschriebenen Verfahren.

## Claims

1. A compound of formula 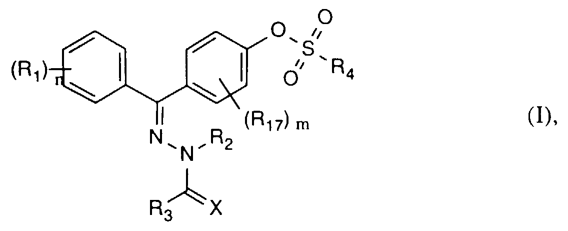 wherein
m is 0, 1, 2, 3 or 4, and, when m is greater than 1, the radicals R₁₇ are identical or different;
n is 0, 1, 2, 3, 4 or 5, and, when n is greater than 1, the radicals R₁ are identical or different;
R₁ and R₁₇ are, each independently of the other, halogen, C₁-C₈alkyl, halo-C₁-C₈alkyl, phenoxy, phenylthio, halo-C₁-C₈alkylthio, phenyl, C₁-C₈alkylthio, C₁-C₈alkylcarbonyl, OSO₂R₄, SO₂NR₁₃R₁₄ or P(=Y)R₂₂R₂₃;
either X is S, R₂ is R₂₁ or H and R₃ is R₃₁, -NR₅R₆ or -OR₂,
or X is O, R₂ is R₂₁ and R₃ is R₃₁,
or X is O, R₂ is H and R₃ is -R₈OR₉, -R₈OCOR₉, -R₈NR₁₀R₁₁ or -R₈R₁₂,
or X and R₃ together are R₁₈;
Y is O or S;
R₄ is C₁-C₈alkyl, halo-C₁-C₈alkyl or halo-C₁-C₈alkoxy;
R₂₁ is C₁-C₈alkyl, C₃-C₆cycloalkyl, halo-C₁-C₈alkyl, C₁-C₄alkoxy-C₁-C₄alkyl, C₁-C₄-alkylthio-C₁-C₄alkyl, R₁₃R₁₄N-C₁-C₄alkyl, C₂-C₈alkenyl, C₂-C₈alkynyl, C₁-C₄acyl, CH=C(CN)₂, or phenyl which is unsubstituted or substituted by from one to five substituents selected from the group consisting of halogen, C₁-C₄alkyl, C₂-C₅alkenyl, halo-C₁-C₄alkyl, C₁-C₄alkoxy, SO₂NR₁₃R₁₄, halo-C₁-C₄alkoxy, C₁-C₄alkylthio, C₁-C₄alkoxy-C₁-C₄alkoxy, halo-C₁-C₄alkylthio, NR₁₃R₁₄, -NO₂ and -CN;
R₃₁ is H, C₁-C₈alkyl, C₃-C₆cycloalkyl, C₃-C₆cycloalkyl-C₁-C₃alkyl, halo-C₁-C₈alkyl, halo-C₃-C₆cycloalkyl-C₁-C₃alkyl, C₂-C₈alkenyl, C₂-C₈alkynyl, halo-C₂-C₈alkenyl, -COOR₇, -SR₂, -R₈OR₉, -R₈OCOR₉, -R₈NR₁₀R₁₁, -R₈R₁₂, -R₈SR_{9,} -R₈SCSR₉, phenyl which is unsubstituted or substituted by from one to five substituents selected from the group consisting of halogen, C₁-C₄alkyl, C₂-C₅alkenyl, halo-C₁-C₄alkyl, halo-C₁-C₄alkoxy, halo-C₁-C₄alkylthio, SO₂NR₁₃R₁₄, C₁-C₄alkoxy, -NO₂ and -CN; or an unsubstituted or substituted, saturated or unsaturated, five- or six-membered heterocyclic or heterobicyclic ring containing from one to three hetero atoms selected from the group O, N and S, the substituents being selected from the group consisting of halogen, C₁-C₄alkyl, halo-C₁-C₄alkyl and C₁-C₄alkoxy;
R₅ and R₆ are each independently of the other H, C₁-C₈alkyl, halo-C₁-C₈alkyl, C₂-C₅alkenyl, C₁-C₄alkoxy, C₂-C₅alkeneoxy or -NR₁₃R₁₄;
R₇ is H, C₁-C₈alkyl, halo-C₁-C₈alkyl or C₂-C₈alkenyl;
R₈ is (CR₁₅R₁₆)ₚ, wherein p is 1, 2, 3 or 4;
R₉ is C₁-C₈alkyl, halo-C₁-C₁₁alkyl, C₃-C₆cycloalkyl, C₂-C₈alkenyl, halo-C₂-C₁₁alkenyl, C₂-C₈alkynyl, NR₁₀R₁₁, OR₁₀, benzyl, or phenyl which is unsubstituted or substituted by from one to five substituents selected from the group consisting of halogen, C₁-C₄alkyl, C₂-C₅alkenyl, halo-C₁-C₄alkyl, C₁-C₄alkoxy, -NO₂ and -CN;
R₁₀ and R₁₁ are each independently of the other C₁-C₈alkyl, C₂-C₈alkenyl or C₁-C₈alkoxy-C₁-C₈alkyl;
R₁₂ is an unsubstituted or substituted, saturated or unsaturated, five- or six-membered heterocyclic or heterobicyclic ring containing from one to three hetero atoms selected from the group O, N and S, the substituents being selected from the group consisting of halogen, C₁-C₄alkyl, halo-C₁-C₄alkyl and C₁-C₄alkoxy;
R₁₃ and R₁₄ are each independently of the other H or C₁-C₈alkyl;
R₁₅ and R₁₆ are each independently of the other H, C₁-C₈alkyl, or phenyl which is unsubstituted or substituted by from one to five substituents selected from the group consisting of halogen, C₁-C₄alkyl, C₂-C₅alkenyl, halo-C₁-C₄alkyl, C₁-C₄alkoxy, -NO₂ and -CN;
R₁₈ is NC(R₁₉)=C(R₁₉)S, NC(R₁₉)=C(R₁₉)O, NC(R₁₉)₂C(R₁₉)₂S, NC(R₁₉)₂C(R₁₉)₂O, NC(R₁₉)=C(R₁₉)C(R₁₉)=N or NC(R₁₉)=NC(R₁₉)=N;
R₁₉ is H, C₁-C₄alkyl, halogen or halo-C₁-C₄alkyl;
R₂₂ is C₁-C₄alkyl, C₁-C₄alkoxy or NR₁₃R₁₄; and
R₂₃ is C₁-C₄alkyl, C₁-C₄alkoxy, C₃-C₄alkylthio or NR₁₃R₁₄,
with the proviso that, in the compounds of formula I, R₂ is other than H when either m is 0, n is 1, R₁ is 4-Cl, R₃ is -N(OCH₃)CH₃, R₄ is CF₃ and X is S, or when in is 0, n is 2, R₁ is 3,4-dichloro, R₃ is -N(OCH₃)CH₃, R₄ is CF₃ and X is S, or when m is 0, n is 1, R₁ is 4-Cl, R₃ is -NHNH₂, R₄ is CF₃ and X is S, or, as the case may be, a tautomer thereof, in each case in free form or in salt form.

2. A compound according to claim 1 of formula I, wherein
m is 0 or 1,
or, as the case may be, a tautomer thereof.

3. A compound according to claim 1 of formula I, wherein
m is 0;
n is 1;
R₁ is fluorine, chlorine or bromine;
R₂ is C₁-C₄alkyl, formyl or acetyl;
R₃ is H, C₁-C₄alkyl, C₃-C₆cycloalkyl, halo-C₁-C₂alkyl, halocyclopropylmethyl, C₂-C₄alkenyl, -COOR₇, -SR₂, -R₈-OCOR₉, -R₈-R₁₂ or an unsubstituted or substituted, unsaturated, five- or six-membered heterocyclic or heterobicyclic ring containing N or S, the substituents being selected from the group fluorine and chlorine;
R₄ is trifluoromethyl;
R₇ is methyl or ethyl;
R₈ is CR₁₅R₁₆;
R₉ is C₁-C₄alkyl, halo-C₈-C₁₁alkyl, cyclopropyl, vinyl, halo-C₈-C₁₁alkenyl, ethynyl or unsubstituted or mono-substituted phenyl, the substituents being selected from the group consisting of chlorine, methyl, methoxy and -NO₂;
R₁₂ is an unsubstituted, unsaturated, five-membered heterocyclic ring containing from one to three hetero atoms selected from the group N and S; and
R₁₅ and R₁₆ are each independently of the other H, methyl or phenyl,
or, as the case may be, a tautomer thereof.

4. A compound according to claim 1 of formula I, wherein
m is 0;
n is 1;
R₁ is fluorine, chlorine or bromine;
R₂ is C₁-C₄alkyl, formyl or acetyl;
R₃ is H, C₁-C₄alkyl, C₃-C₆cycloalkyl, halo-C₁-C₂alkyl, halocyclopropylmethyl, C₂-C₄alkenyl, -COOR₇, -SR₂, -R₈-OCOR₉, -R₈-R₁₂ or an unsubstituted or substituted, unsaturated, five- or six-membered heterocyclic or heterobicyclic ring containing N or S, the substituents being selected from the group fluorine and chlorine;
R₄ is trifluoromethyl;
R₇ is methyl or ethyl;
R₈ is CH₂, C(methyl)₂ or CH(phenyl);
R₉ is C₁-C₄alkyl, halo-C₈-C₁₁alkyl, cyclopropyl, vinyl, halo-C₈-C₁₁alkenyl, ethynyl or unsubstituted or mono-substituted phenyl, the substituents being selected from the group consisting of chlorine, methyl, methoxy and -NO₂; and
R₁₂ is an unsubstituted, unsaturated, five-membered heterocyclic ring containing from one to three hetero atoms selected from the group N and S,
or, as the case may be, a tautomer thereof.

5. 4-Chloro-4'-trifluoromethanesulfonyloxybenzophenone-N-formyl-N-methylhydrazone according to claim 1.

6. A process for the preparation of a compound according to claim 1 of formula I or, as the case may be, a tautomer thereof, in each case in free form or in salt form, taking into account the proviso mentioned above, in which process
a) a compound of formula 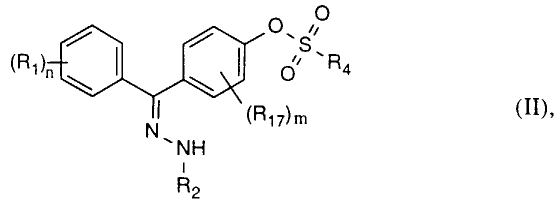 which is known or can be prepared analogously to corresponding known compounds and wherein m, n, R₁, R₂, R₄ and R₁₇ are as defined for formula I, or, as the case may be, a tautomer thereof, in free form or in salt form, is reacted with phosgene or thiophosgene, preferably in the presence of a basel and the intermediate, which is optionally isolated, is reacted, if desired in the presence of a base, with a compound of formula Y₁H, wherein Y₁ is -NR₅R₆, -OR₂ or -SR₂ and R₂, R₅ and R₆ are as defined for formula I, or with a salt thereof, or
b) for the preparation of a compound of formula I wherein X is O, a compound of formula II is reacted, if desired in the presence of a base, with a compound of formula R₃COY₂, wherein R₃ is as defined for formula I with the exception of the meanings -OR₂ and -SR₂, and Y₂ is a leaving group, or
c) for the preparation of a compound of formula I wherein X is S, a compound of formula II is reacted with a compound of formula Y₃-N=C=S, wherein Y₃ is H, C₁-C₈alkyl, halo-C₁-C₈alkyl or C₂-C₅alkenyl, preferably H or C₁-C₈alkyl, to form compounds of formula I wherein X is S, or
d) for the preparation of a compound of formula I wherein X is S and R₃ is -SR, wherein R is C₁-C₈alkyl, C₃-C₆cycloalkyl, C₂-C₈alkenyl or C₂-C₈alkynyl, preferably C₁-C₈alkyl or C₃-C₆cycloalkyl, especially C₁-C₈alkyl, a compound of formula II is reacted, if desired in the presence of a base, with CS₂, and the intermediate, which is optionally isolated, is reacted, if desired in the presence of a base, with a compound of formula RY₄, wherein R is as defined above and Y₄ is a leaving group, or
e) for the preparation of a compound of formula I wherein X is O, a compound of formula II is reacted, if desired in the presence of a base, with a compound of formula CIR₈COY₅, wherein R₈ is as defined for formula I and Y₅ is a leaving group, and the resulting intermediate is reacted, if desired in the presence of a base, optionally in the presence of an alkali metal halide, such as in the presence of sodium iodide, with a compound of formula R₉COOH, HNR₁₀R₁₁ or R₁₂, wherein R₉, R₁₀, R₁₁ and R₁₂ are as defined for formula I, or
f) for the preparation of a compound of formula I wherein X is S, a compound of formula 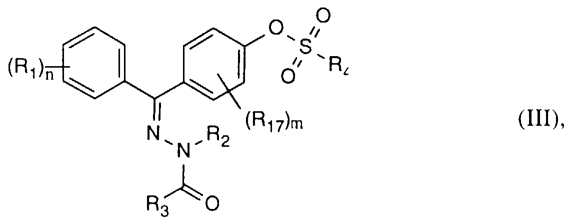 which is known or can be prepared analogously to corresponding known compounds, for example in accordance with Variant b), and wherein m, n, R₁, R₂, R₃, R₄ and R₁₇ are as defined for formula I, is reacted with P₂S₅, or
g) for the preparation of a compound of formula I wherein X is S and R₂ is H, a compound of formula 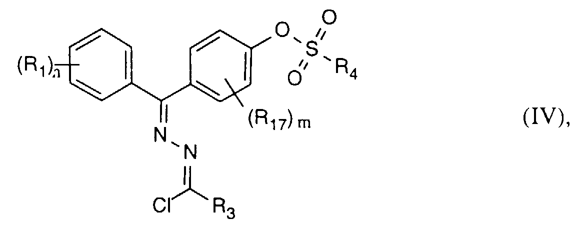 which is known or can be prepared analogously to corresponding known compounds and wherein m, n, R₁, R₃, R₄ and R₁₇ are as defined for formula I, is reacted, if desired in the presence of a base, with H₂S, or
h) a compound of formula I wherein R₂ is H is reacted, if desired in the presence of a base, with a compound of formula R₂Y₆, wherein R₂ is as defined for formula I with the exception of the meaning H, and Y₆ is a leaving group, or
i) a compound of formula 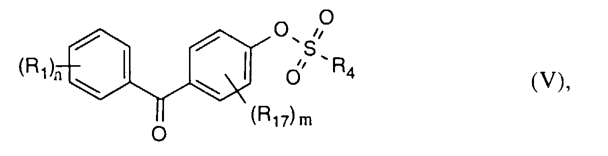 which is known or can be prepared analogously to corresponding known compounds and wherein m, n, R₁, R₄ and R₁₇ are as defined for formula I, is reacted, if desired in the presence of an acid catalyst, if desired in the presence of a water-binding agent, with a compound of formula 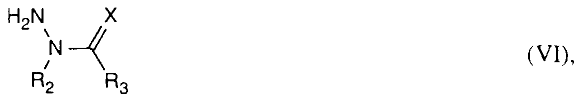 which is known or can be prepared analogously to corresponding known compounds and wherein R₂, R₃ and X are as defined for formula I, or with a salt thereof and/or, as the case may be, with a tautomer thereof,
and in each case, if desired, a compound of formula I obtainable according to the process or by other means, or a tautomer thereof, in each case in free form or in salt form, is converted into a different compound of formula I or a tautomer thereof, a mixture of isomers obtainable according to the process is separated and the desired isomer is isolated and/or a free compound of formula I obtainable according to the process, or a tautomer thereof, is converted into a salt, or a salt of a compound of formula I or of a tautomer thereof obtainable according to the process is converted into the free compound of formula I or a tautomer thereof or into a different salt.

7. A pesticidal composition which comprises at least one compound according to claim 1 of formula I or, as the case may be, a tautomer thereof, in each case in free form or in the form of an agrochemically acceptable salt, as active ingredient and, where appropriate, at least one adjuvant.

8. A process for the preparation of a composition according to claim 7 comprising at least one adjuvant, which process comprises homogeneously mixing and/or grinding the active ingredient with the adjuvant(s).

9. The use of a compound according to claim 1 of formula I or, as the case may be, a tautomer thereof, in each case in free form or in the form of an agrochemically acceptable salt, in the preparation of a composition according to claim 7.

10. The use of a composition according to claim 7 in the control of pests.

11. The use according to claim 10 in the protection of plant propagation material.

12. A method of controlling pests, which comprises applying to the pests or their habitat a composition according to claim 7.

13. A method according to claim 12 for the protection of plant propagation material, which comprises treating the propagation material or the planting site of the propagation material.

14. Plant propagation material treated in accordance with the method described in claim 13.

## Revendications

1. Composé de formule : 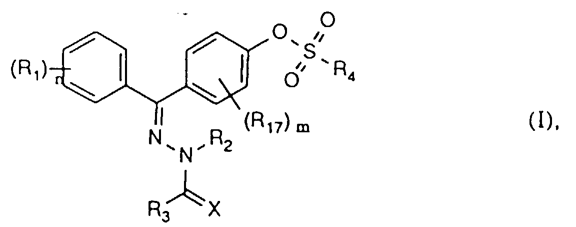 dans laquelle :
m vaut 0, 1, 2, 3 ou 4, auquel cas, quand m est supérieur à 1, les radicaux R₁₇ sont identiques ou différents ;
n vaut 0, 1, 2, 3, 4 ou 5, auquel cas, quand n est supérieur à 1, les radicaux R₁ sont identiques ou différents ;
R₁ et R₁₇ sont chacun indépendamment de l'autre un atome d'halogène, un groupe alkyle en C₁-C₈, halogéno (alkyle en C₁-C₈), phénoxy, phénylthio, halogéno-(alkylthio en C₁-C₈), phényle, alkylthio en C₁-C₈, (alkyle en C₁-C₈)carbonyle, OSO₂R₄, SO₂NR₁₃R₁₄, ou P(=Y)R₂₂R₂₃ ;
ou bien X est S, R₂ est R₂₁, ou encore H et R₃ sont R₃₁, -NR₅R₆ ou -OR₂, ou bien X est O, R₂ est R₂₁ et R₃ est R₃₁,
ou bien X est O, R₂ est H et R₃ est -R₈OR₉, -R₈OCOR₉, -R₈NR₁₀R₁₁ ou -R₈R₁₂,
ou bien X et R₃ forment ensemble R₁₈ ;
Y est O ou S ;
R₄ est un groupe alkyle en C₁-C₈, halogéno(alkyle en C₁-C₈) ou halogéno(alcoxy en C₁-C₈) ;
R₂₁ est un groupe alkyle en C₁-C₈, cycloalkyle en C₃-C₆, halogéno(alkyle en C₁-C₈), (alcoxy en C₁-C₄)-(alkyle en C₁-C₄), (alkylthio en C₁-C₄)-(alkyle en C₁-C₄), R₁₃R₁₄N-(alkyle en C₁-C₄), alcényle en C₂-C₈, alcynyle en C₂-C₈, acyle en C₁-C₄, CH=C(CN)₂, ou phényle non substitué ou une à cinq fois substitué, auquel cas les substituants sont choisis parmi l'ensemble comprenant les atomes d'halogène, les groupes alkyle en C₁-C₄, alcényle en C₂-C₅, halogéno(alkyle en C₁-C₄), alcoxy en C₁-C₄, SONR₁₃R₁₄, halogéno-(alcoxy en C₁-C₄), alkylthio en C₁-C₄, (alcoxy en C₁-C₄)-(alcoxy en C₁-C₄), halogéno-(alkylthio en C₁-C₄), NR₁₃R₁₄, -NO₂ et -CN ;
R₃₁ est un atome d'hydrogène ou un groupe alkyle en C₁-C₈, cycloalkyle en C₃-C₆, (cycloalkyle en C₃-C₆)-(alkyle en C₁-C₃), halogéno-(alkyle en C₁-C₈), halogéno-(cycloalkyle en C₃-C₆)-(alkyle en C₁-C₃), alcényle en C₂-C₈, alcynyle en C₂-C₈, halogéno-(alcényle en C₂-C₈), -COOR₇, -SR₂, -R₈OR₉, -R₈OCOR₉, -R₈NR₁₀R₁₁, -R₈R₁₂, -R₈SR₉, -R₈SCSR₉, phényle non substitué ou encore une à cinq fois substitué, auquel cas les substituants sont choisis parmi l'ensemble comprenant les atomes d'halogène, les groupes alkyle en C₁-C₄, alcényle en C₂-C₅, halogéno-(alkyle en C₁-C₄), halogéno-(alcoxy en C₁-C₄), halogéno-(alkylthio en C₁-C₄), SONR₁₃R₁₄, alcoxy en C₁-C₄, -NO₂ et -CN ; un noyau hétérocyclique ou hétérobicyclique à 5 ou 6 chaînons, non substitué ou substitué, saturé ou insaturé, contenant de 1 à 3 hétéroatomes choisis parmi ensemble comprenant, O, N et S, auquel cas les substituants sont choisis parmi l'ensemble comprenant les atomes d'halogène, les groupes alkyle en C₁-C₄, halogéno-(alkyle en C₁-C₄) et (alcoxy en C₁-C₄) ;
R₅ et R₆ sont chacun indépendamment de l'autre un atome d'hydrogène ou un groupe alkyle en C₁-C₈, halogéno(alkyle en C₁-C₈), alcényle en C₂-C₅, alcoxy en C₁-C₄, alcénoxy en C₂-C₅, ou -NR₁₃R₁₄ ;
R₇ est un atome d'hydrogène ou un groupe alkyle en C₁-C₈, halogéno-(alkyle en C₁-C₈) ou alcényle en C₂-C₈ ;
R₈ est (CR₁₅R₁₆)_{p,} où p vaut 1, 2, 3 ou 4 ;
R₉ est un groupe alkyle en C₁-C₈, halogéno-(alkyle en C₁-C₁₁), cycloalkyle en C₃-C₆, alcényle en C₂-C₈, halogéno(alcényle en C₂-C₁₁), alcynyle en C₂-C₈, NR₁₀R₁₁, OR₁₀, benzyle, ou phényle non substitué ou une à cinq fois substitué, auquel cas les substituants sont choisis parmi l'ensemble comprenant les atomes d'halogène et les groupes alkyle en C₁-C₄, alcényle en C₂-C₅, halogéno-(alkyle en C₁-C₄), alcoxy en C₁-C₄, -NO₂ et -CN ;
R₁₀ et R₁₁ sont chacun indépendamment de l'autre un groupe alkyle en C₁-C₈, alcényle en C₂-C₈, ou (alcoxy en C₁-C₈)-(alkyle en C₁-C₈) ;
R₁₂ est un noyau hétérocyclique ou hétérobicyclique à 5 ou 6 chaînons, non substitué ou substitué, saturé ou insaturé, contenant de 1 à 3 hétéroatomes choisis parmi l'ensemble comprenant O, N et S, auquel cas les substituants sont choisis parmi l'ensemble comprenant les atomes d'halogène et les groupes alkyle en C₁-C₄, halogéno(alkyle en C₁-C₄), alcoxy en C₁-C₄ ;
R₁₃ et R₁₄ sont chacun indépendamment de l'autre un atome d'hydrogène ou un groupe alkyle en C₁-C₈ ;
R₁₅ et R₁₆ sont chacun indépendamment de l'autre un atome d'hydrogène, un groupe alkyle en C₁-C₈, ou phényle non substitué ou une à cinq fois substitué, auquel cas les substituants sont choisis parmi l'ensemble comprenant les atomes d'halogène et les groupes alkyle en C₁-C₄, alcényle en C₂-C₅, halogéno-(alkyle en C₁-C₄), alcoxy en C₁-C₄, -NO₂ et -CN ;
R₁₈ est NC(R₁₉)=C(R₁₉)S, NC(R₁₉)=C(R₁₉)O, NC(R₁₉)₂S, NC(R₁₉)₂C(R₁₉)₂O, NC(R₁₉)=C(R₁₉)C(R₁₉)=N ou NC(R₁₉)=NC(R₁₉)=N ;
R₁₉ est un atome d'hydrogène ou un groupe alkyle en C₁-C₄, halogéno ou halogéno-(alkyle en C₁-C₄) ;
R₂₂ est un groupe alkyle en C₁-C₄, alcoxy en C₁-C₄ ou NR₁₃R₁₄ **;** et
R₂₃ est un groupe alkyle en C₁-C₄, alcoxy en C₁-C₄, alkylthio en C₃-C₄, ou NR₁₃R₁₄,
du moment que, dans les composés de formule I, R₂ n'est pas différent de H, quand ou bien m vaut 0, n vaut 1, R₁ est 4-Cl, R₃ est -N(OCH₃)CH₃, R₄ est CF₃ et X est S, ou bien m vaut 0, n vaut 2, R₁ est 3, 4-dichloro, R₃ estN(OCH₃)CH₃, R₄ est CF₃ et X est S, ou bien quand m vaut 0, n vaut 1, R₁ est 4-Cl, R₃ est -NHNH₂, R₄ est CF₃ et X est S, et éventuellement un de ses tautomères, chaque fois sous forme libre ou sous forme d'un sel.

2. Composé selon la revendication 1 de formule I, dans laquelle m vaut 0 ou 1, ou éventuellement l'un de ses tautomères.

3. Composé selon la revendication 1 de formule I, dans laquelle :
m vaut 0 ;
n vaut 1 ;
R₁ est un atome de fluor, de chlore ou de brome ;
R₂ est un groupe alkyle en C₁-C₄, formyle ou acétyle ;
R₃ est un atome d'hydrogène ou un groupe alkyle en C₁-C₄, cycloalkyle en C₃-C₆, halogéno-(alkyle en C₁-C₂), halogéno-(cyclopropylméthyle), alcényle en C₂-C₄, -COOR₇, -SR₂, -R₈-OCOR₉, -R₈-R₁₂, ou un noyau hétérocyclique ou hétérobicyclique à 5 ou 6 chaînons, insaturé, non substitué ou substitué, contenant N ou S, les substituants étant choisis parmi le fluor et le chlore ;
R₄ est le groupe trifluorométhyle ;
R₇ est le groupe méthyle ou éthyle ;
R₈ est CR₁₅R₁₆ ;
R₉ est un groupe alkyle en C₁-C₄, halogéno-(alkyle en C₈-C₁₁), cyclopropyle, vinyle, halogéno-(alcényle en C₈-C₁₁), éthynyle, ou phényle non substitué ou mono substitué, les substituants étant choisis parmi l'ensemble comprenant le chlore et les groupes méthyle, méthoxy et -NO₂ ;
R₁₂ est un noyau hétérocyclique insaturé à 5 chaînons et non substitué contenant de 1 à 3 hétéroatomes choisis parmi N et S ; et
R₁₅ et R₁₆ sont chacun indépendamment de l'autre un atome d'hydrogène ou un groupe méthyle ou phényle,
ou éventuellement l'un de ses tautomères.

4. Composé selon la revendication 1 de formule I, dans laquelle :
m vaut 0 ;
n vaut 1 ;
R₁ est un atome de fluor, de chlore ou de brome ;
R₂ est un groupe alkyle en C₁-C₄, formyle ou acétyle ;
R₃ est un atome d'hydrogène ou un groupe alkyle en C₁-C₄, cycloalkyle en C₃-C₆, halogéno-(alkyle en C₁-C₂), halogéno-(cyclopropylméthyle), alcényle en C₂-C₄, -COOR₇, -SR₂, -R₈-OCOR₉, -R₈-R₁₂, ou un noyau hétérocyclique ou hétérobicyclique à 5 ou 6 chaînons, insaturé, non substitué ou substitué, contenant N ou S, les substituants étant choisis parmi le fluor et le chlore ;
R₄ est le groupe trifluorométhyle ;
R₇ est le groupe méthyle ou éthyle ;
R₈ est CH2 ou un groupe C(méthyl)₂ ou CH(phényle)
R₉ est un groupe alkyle en C₁-C₄, halogéno-(alkyle en C₈-C₁₁), cyclopropyle, vinyle, halogéno-(alcényle en C₈-C₁₁), éthynyle, ou phényle non substitué ou mono substitué, les substituants étant choisis parmi l'ensemble comprenant le chlore et les groupes méthyle, méthoxy et -NO₂ ; et
R₁₂ est un noyau hétérocyclique insaturé à 5 chaînons et non substitué contenant de 1 à 3 hétéroatomes choisis parmi N et S.

5. 4-Chloro-4'-trifluorométhanesulfonyloxybenzophénone-N-formyl-N-méthylhydrazone selon la revendication 1.

6. Procédé de préparation d'un composé selon la revendication 1 de formule I ou éventuellement l'un de ses tautomères, dans chaque cas sous forme libre ou sous forme d'un sel, compte tenu de la condition mentionnée ci-dessus, caractérisé en ce que :
(a) on fait réagir avec du phosgène ou du thiophosgène, de préférence en présence d'une base, un composé de formule : 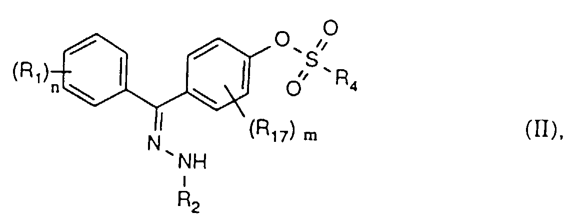 qui est connu ou peut être préparé par analogie avec des composés connus correspondants, et où m, n, R₁, R₂, R₄ et R₁₇ ont les significations données pour la formule I, ou éventuellement l'un de ses tautomères, sous forme libre ou sous forme d'un sel, et on fait réagir le produit intermédiaire éventuellement isolé, éventuellement en présence d'une base, avec un composé de formule Y₁H dans laquelle Y₁ est -NR₅R₆, -OR₂ ou -SR₂, et R₂, R₅ et R₆ ont les significations données pour la formule I, ou un de ses sels, ou bien
(b) pour préparer un composé de formule I dans laquelle X est O, on fait réagir un composé de formule II, éventuellement en présence d'une base, avec un composé de formule R₃COY₂ dans laquelle R₃ a les significations données pour la formule I, à l'exception de -OR₂ et -SR₂, et Y₂ est un groupe éliminable, ou bien
(c) pour préparer un composé de formule I dans laquelle X est S, on fait réagir un composé de formule II avec un composé de formule Y₃-N=C=S, dans laquelle Y₃ est H, un groupe alkyle en C₁-C₈, halogéno-(alkyle en C₁-C₈) ou alcényle en C₂-C₅, de préférence H ou alkyle en C₁-C₈, pour obtenir des composés de formule I dans laquelle X est S, ou bien
(d) pour préparer un composé de formule I dans laquelle X est S et R₃ est -SR, où R est un groupe alkyle en C₁-C₈, cycloalkyle en C₃-C₆, alcényle en C₂-C₈ ou alcynyle en C₂-C₈, de préférence alkyle en C₁-C₈, cycloalkyle en C₃-C₆, en particulier alkyle en C₁-C₈, on fait réagir avec du CS₂ un composé de formule II, éventuellement en présence d'une base, et on fait réagir le produit intermédiaire éventuellement isolé, éventuellement en présence d'une base, avec un composé de formule RY₄ dans laquelle R a les significations données ci-dessus et Y est un groupe éliminable, ou bien
(e) pour préparer un composé de formule I dans laquelle X est O, on fait réagir un composé de formule II, éventuellement en présence d'une base, avec un composé de formule CIR₈COY₅, dans laquelle R₈ a les significations données pour la formule I et Y₅ est un groupe éliminable, et on fait réagir le produit intermédiaire ainsi obtenu, éventuellement en présence d'une base, éventuellement en présence d'un halogénure d'un métal alcalin, par exemple en présence d'iodure de sodium, avec un composé de formule R₉COOH, HNR₁₀R₁₁ ou R₁₂, où R₉, R₁₀, R₁₁ et R₁₂ ont les significations données pour la formule I, ou bien
(f) pour préparer un composé de formule I dans laquelle X est S, on fait réagir avec du P₂S₅ un composé de formule : 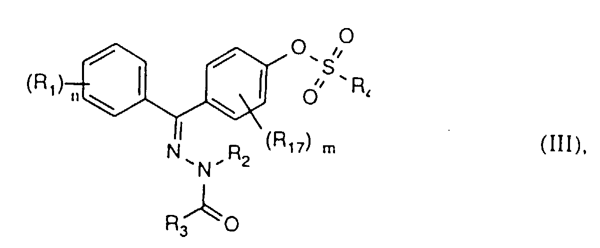 qui est connu ou peut être préparé par analogie avec des composés connus correspondants, par exemple d'après la variante b), et où m, n, R₁, R₂, R₃ et R₁₇ ont les significations données pour la formule I, ou bien
(g) pour préparer un composé de formule I dans laquelle X est S et R₂ est H, on fait réagir avec du H₂S un composé de formule : 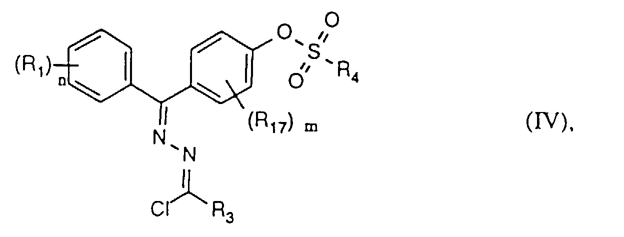 qui est connu ou peut être préparé par analogie avec des composés connus correspondants, et où m, n, R₁, R₃, R₄ et R₁₇ ont les significations données pour la formule I, éventuellement en présence d'une base, ou bien
(h) on fait réagir un composé de formule I dans laquelle R₂ est H, éventuellement en présence d'une base, avec un composé de formule R₂Y₆ dans laquelle R₂ a les significations données pour la formule I à l'exception de H, et Y₆ est un groupe éliminable, ou bien
(i) on fait réagir un composé de formule : 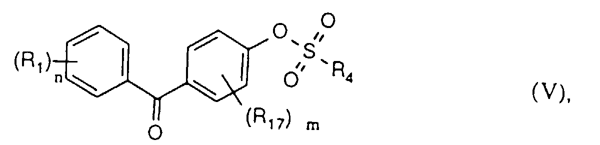 qui est connu ou peut être préparé par analogie avec des composés connus correspondants, et où m, n, R₁, R₄ et R₁₇ ont les significations données pour la formule I, éventuellement en présence d'un catalyseur acide, éventuellement en présence d'un agent fixant l'eau, avec un composé de formule : 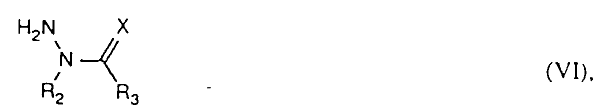 qui est connu ou peut être préparé par analogie avec des composés connus correspondants et où R₂, R₃ et Y ont les significations données pour la formule I, ou l'un de ses sels et/ou éventuellement l'un de ses tautomères, et chaque fois que l'on souhaite, on convertit un composé de formule I ou l'un de ses tautomères, sous forme libre ou sous forme d'un sel, pouvant être obtenu selon l'invention ou d'une autre manière, en un autre composé de formule I ou l'un de ses tautomères, on sépare le mélange d'isomères pouvant être obtenu selon l'invention et on isole l'isomère recherché, et/ou on convertit un composé libre de formule I ou l'un de ses tautomères, pouvant être obtenu selon l'invention, en un sel, ou encore on convertit un sel, pouvant être obtenu selon l'invention, d'un composé de formule I ou d'un de ses tautomères, en le composé libre de formule I ou l'un de ses tautomères, ou en un autre sel.

7. Pesticide, caractérisé en ce qu'il contient au moins un composé selon la revendication 1 de formule I ou éventuellement l'un de ses tautomères, dans chaque cas sous forme libre ou sous forme d'un sel utilisable en agrochimie, en tant que matière active, et éventuellement au moins un adjuvant.

8. Procédé de préparation d'un pesticide contenant au moins un adjuvant selon la revendication 7, caractérisé en ce qu'on mélange intimement et/ou broie la matière active et le ou les adjuvants.

9. Utilisation d'un composé selon la revendication 1 de formule I ou éventuellement d'un de ses tautomères, dans chaque cas sous forme libre ou sous forme d'un sel utilisable en agrochimie, pour préparer un pesticide selon la revendication 7.

10. Utilisation d'un pesticide selon la revendication 7, pour maîtriser les ravageurs.

11. Utilisation selon la revendication 40, pour protéger les organes de multiplication végétative.

12. Procédé pour maîtriser les ravageurs, caractérisé en ce qu'on applique un pesticide selon la revendication 7 sur les ravageurs ou dans leur espace vital.

13. Procédé selon la revendication 12, pour protéger les organes de multiplication végétative, caractérisé en ce qu'on traite les organes de multiplication ou le site d'application des organes de multiplication.

14. Organe de multiplication végétative, traité par le procédé décrit dans la revendication 13.
